# EUROPEAN PATENT SPECIFICATION

(11) **EP 2 429 477 B1**
(45) Date of publication and mention of the grant of the patent: **10.07.2013**
(21) Application number: 10728449.9
(22) Date of filing: 13.05.2010
(51) Int. Cl.: A61H 31/00, A61H 23/04, A61B 17/135, A41D 13/00

(54) **PERFORMANCE ENHANCEMENT**
LEISTUNGSVERSTÄRKUNG
AMELIORATION DE LA PERFORMANCE

(30) Priority: 13.05.2009 US 177970 P
(43) Date of publication of application: 21.03.2012
(73) Proprietor: The Hospital For Sick Children, Toronto, Ontario M5G 1X8 (CA); Cellaegis Devices Inc., Toronto, ON M6N 1G9 (CA)
(72) Inventor: REDINGTON, Andrew, Toronto, Ontario M4V 2R5 (CA); CALDARONE, Christopher, Toronto, Ontario M4W 2A6 (CA); GANSKE, Rocky, Oakville, Ontario L6L 6V8 (CA)
(74) Representative: HOFFMANN EITLE
(86) International application number: PCT/US2010/001424
(87) International publication number: WO 2010/132115

(56) References cited:
- WO-A1-2008/148045
- US-A1- 2005 070 405
- US-A1- 2008 139 949
- US-A1- 2008 222 769
- US-A1- 2009 137 884

## Description

### BACKGROUND OF THE INVENTION

Limitation of exercise capacity in health and disease is due to the inability of the heart and circulation to provide enough oxygen, and other nutrients in the blood, for the muscles to generate energy and work. As the balance between blood flow (too low) or requirements for oxygen and nutrients (too high) develops during exercise, then the muscles work less efficiently, there is a build up of acid in the tissues, the individual becomes fatigued, and exercise stops. The only known natural method to increase exercise performance is through repetitive exercise. Repetitive exercise has been documented to improve exercise performance in athletes, healthy non-athletes, and those with cardiovascular disease.

### SUMMARY OF THE INVENTION

The invention relates to the surprising and unexpected discovery that it is possible to improve physical performance without repetitive exercise. In particular, it has been unexpectedly found that subjecting an individual to transient ischemic events enhances the performance of physical activity such as but not limited to competitive athletic performance. This was surprising, at least in part, because such deliberately induced transient ischemic events heretofore had only been contemplated for use in subjects who were intended to undergo surgical procedures (and would thereby be experiencing ischemia during the surgical procedure) or subjects who, due to their present status or medical history (including familial history), were expected to undergo an ischemic event (such as a heart attack). These deliberate transient ischemic events are followed by periods of time in which blood is allowed to flow back into the tissue (referred to herein as reperfusion). This two step process may be repeated a number of times. Whether a single cycle or repeated cycles are used, this regimen is referred to herein as remote ischemic preconditioning or RIPC.

The invention therefore provides in one aspect a method for enhancing physical performance comprising performing a remote ischemic preconditioning regimen on a subject prior to physical activity.

In one embodiment, the physical activity is maximal physical activity. In another embodiment, the physical activity is submaximal physical activity.

In one embodiment, the remote ischemic preconditioning regimen is performed within 24 hours of the physical activity. In another embodiment, the remote ischemic preconditioning regimen is performed within 2 hours of the physical activity. In another embodiment, the remote ischemic preconditioning regimen is performed within 20 minutes of the physical activity.

In one embodiment, the remote ischemic preconditioning regimen comprises 1, 2, 3, 4 or 5 cycles of supra-systolic pressure and reperfusion.

In one embodiment, the remote ischemic preconditioning regimen comprises at least four cycles of supra-systolic pressure and reperfusion.

In one embodiment, the remote ischemic preconditioning regimen comprises more than 5 cycles of supra-systolic pressure and reperfusion.

In one embodiment, the remote ischemic preconditioning regimen comprises one or more cycles comprising about 1 minute up to 20 minutes of supra-systolic pressure.

In one embodiment, the remote ischemic preconditioning regimen comprises one or more cycles comprising about 1 minute, about 2 minutes, about 3 minutes, about 4 minutes, or about 5 minutes of supra-systolic pressure.

In one embodiment, the remote ischemic preconditioning regimen comprises one or more cycles comprising about 1 minute up to 20 minutes of reperfusion.

In one embodiment, the remote ischemic preconditioning regimen comprises one or more cycles comprising about 1 minute, about 2 minutes, about 3 minutes, about 4 minutes, or about 5 minutes of reperfusion.

In one embodiment, the remote ischemic preconditioning regimen comprises one or more cycles of 5 minutes of supra-systolic pressure and 5 minutes of reperfusion. In one embodiment, the remote ischemic preconditioning regimen comprises 4 cycles of 5 minutes of supra-systolic pressures and 5 minutes of reperfusion.

In one embodiment, the supra-systolic pressure is a pressure that is at least 15 mmHg above systolic pressure. In one embodiment, the supra-systolic pressure is about 200 mmHg.

In one embodiment, the remote ischemic preconditioning regimen is performed on an upper limb. In one embodiment, the remote ischemic preconditioning is performed on a lower limb. In one embodiment, the remote ischemic preconditioning regimen is performed at two or more locations on the body, whether in a simultaneous, overlapping, or alternating manner.

In another embodiment, the subject does not experience an increase in lactate production as a result of the maximal physical activity.

**In one embodiment, the subject is human. In other embodiments, the subject is a non-human including but not limited to a horse or a dog.**

In one embodiment, the method causes an improvement in physical activity in the range of about 1-20%, 1-10%, or 1-5%. In one embodiment, the method causes about a 1.5% improvement in physical activity. In other embodiments, the method causes about a 0.5%, about a 0.6%, about a 0.7%, about a 0.8%, about a 0.9%, about a 1.0%, about a 1.1%, about a 1.2%, about a 1.3%, or about a 1.4% improvement in physical activity.

In another aspect, the invention provides a system comprising a cuff configured to retract about a remote location of a subject; an actuator connected to the cuff, that when actuated causes the cuff to contract about the remote location of the subject to reduce blood flow there through; and a controller that controls the actuator to operate according to a treatment protocol that includes one or a plurality of sequentially actuated treatment cycles, each treatment cycle comprising cuff actuation, during which the actuator contracts the cuff about the remote location of the subject to a pressure above systolic pressure to occlude blood flow through the remote location; an ischemic duration, during which the actuator maintains the cuff contracted about the remote location at a set point above systolic pressure to occlude blood flow through the remote location, the ischemic duration lasting for a period of time ranging from about 1 minute to 20 minutes; cuff release, during which the actuator releases the cuff to allow blood flow through the remote location; and a reperfusion duration, during which the cuff is maintained in an at least partially relaxed state to allow blood flow through the remote location, the reperfusion duration lasting for a period of time ranging from about 1 minute to 20 minutes, wherein the system, when in use, is comprised in a water-resistant housing, and/or is attached in whole or in part (e.g., at least the cuff element) to a garment.

In one embodiment, the system is comprised within a garment (e.g., between layers of the garment). In one embodiment, garment is athletic apparel. In one embodiment, the system comprises a strap, harness or belt.

In one embodiment, the controller is a remote (or wireless) controller.

In another aspect, the invention provides a device comprising a contractable cuff for use in enhancing physical performance, comprising performing a remote ischemic preconditioning regimen on a healthy subject prior to a maximal physical activity by the subject using the device.

In another aspect, the invention provides for use of a device comprising a contractable cuff for enhancing physical performance, comprising using the device to perform a remote ischemic preconditioning regimen on a healthy subject prior to a maximal physical activity by the subject.

In another aspect, the invention provides a device comprising a contractable cuff for use in enhancing physical activity, comprising performing a remote ischemic preconditioning regimen on a subject having a cardiovascular condition prior to a physical activity, using the device.

In another aspect, the invention provides for use of a device comprising a contractable cuff for enhancing physical activity, comprising using the device to perform a remote ischemic preconditioning regimen on a subject having a cardiovascular condition prior to a physical activity.

The device and the use of the device in performing a remote ischemic preconditioning regimen enhances performance of the physical activity by the subject.

In one embodiment, the device is a manual device. In one embodiment, the device is an automatic device.

In one embodiment, the contractable cuff is an inflatable cuff.

In one embodiment, the device comprises a strap, harness or belt.

In one embodiment, the device comprises an actuator and a controller. In one embodiment, the device comprises a cuff configured to retract about a remote location of a subject; an actuator connected to the cuff and that, when actuated, causes the cuff to contract about the remote location of the subject to reduce blood flow there through; and a controller that controls the actuator. In one embodiment, the controller is a remote (or wireless) controller. In one embodiment, the controller controls the actuator to operate according to a treatment protocol that includes one or a plurality of sequentially actuated treatment cycles, each treatment cycle comprising cuff actuation, during which the actuator contracts the cuff about the remote location of the subject to a pressure above systolic pressure to occlude blood flow through the remote location; an ischemic duration, during which the actuator maintains the cuff contracted about the remote location at a set point above systolic pressure to occlude blood flow through the remote location; cuff release, during which the actuator releases the cuff to allow blood flow through the remote location; and a reperfusion duration, during which the cuff is maintained in an at least partially relaxed state to allow blood flow through the remote location.

In one embodiment, the ischemic duration lasts for about 1 minute up to 20 minutes. In one embodiment, the reperfusion duration lasts for about 1 minute up to 20 minutes.

In one embodiment, the remote location is a limb.

In another aspect, the invention provides a garment comprising a cuff configured to retract about a remote location of a subject, wherein the garment is an athletic garment.

In one embodiment, the garment comprises two or more cuffs, each configured to retract about a remote location of a subject.

In one embodiment, the garment is a swimsuit. In one embodiment, the garment is a running suit.

In one embodiment, the cuff is located on an inner surface of the garment. In one embodiment, the cuff is located between layers of the garment.

In one embodiment, the remote location is a limb. In one embodiment, the limb is an upper limb. In one embodiment, the limb is a lower limb.

In one embodiment, the garment further comprises an actuator connected to the cuff, that when actuated causes the cuff to contract about the remote location of the subject.

In one embodiment, the garment further comprises a controller that controls the actuator. In one embodiment, the controller controls the actuator to operate according to a treatment protocol that includes one or a plurality of sequentially actuated treatment cycles, each treatment cycle comprising cuff actuation, during which the actuator contracts the cuff about the remote location of the subject to a pressure above systolic pressure to occlude blood flow through the remote location; an ischemic duration, during which the actuator maintains the cuff contracted about the remote location at a set point above systolic pressure to occlude blood flow through the remote location, the ischemic duration lasting for a period of time ranging from about 1 minute to 20 minutes; cuff release, during which the actuator releases the cuff to allow blood flow through the remote location; and a reperfusion duration, during which the cuff is maintained in an at least partially relaxed state to allow blood flow through the remote location, the reperfusion duration lasting for a period of time ranging from about 1 minute to 20 minutes.

These and other aspects and embodiments of the invention will be discussed in greater detail herein.

### BRIEF DESCRIPTION OF THE FIGURES

The accompanying drawings are not intended to be drawn to scale. In the drawings, each identical or nearly identical component that is illustrated in various figures is represented by a like numeral. For purposes of clarity, not every component may be labeled in every drawing.

Various embodiments of the invention will now be described, by way of example, with reference to the accompanying drawings, in which:
FIG. 1 is a schematic representation of one embodiment of a remote ischemic preconditioning system, including a pneumatically inflatable cuff configured to contract about the limb of a subject.
FIG. 2 is a block diagram of one embodiment of an operating scheme of the RIPC system.
FIG. 3 shows an alternate embodiment of a cuff configured to contract about the limb of a subject.
FIG. 4 is a flow diagram for submaximal exercise testing.
FIG. 5 is a flow diagram for maximal exercise testing.

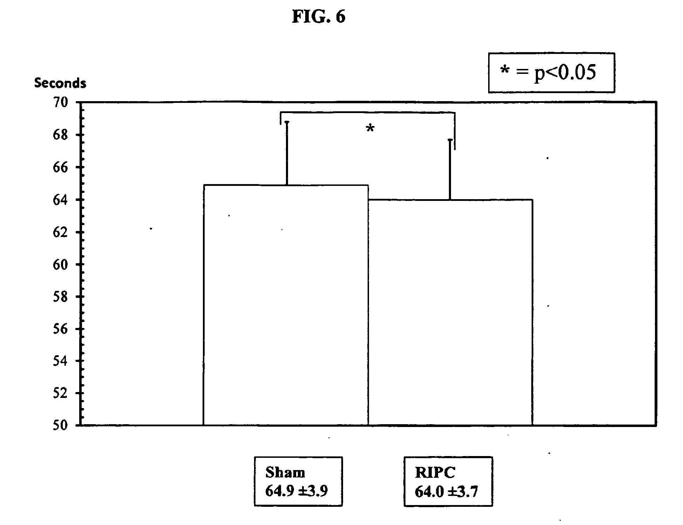

FIG. 6 is a histogram showing 100 meter swim times at maximal effort for control and test groups.
FIG. 7 is a flow diagram for submaximal exercise testing.
FIG. 8 is a flow diagram for maximal exercise testing.
FIG. 9 is a graph showing the effect of RIPC on maximal swim time. Value expressed in seconds. Each black line represents different elite swimmers. N = 18 for all groups.
FIG. 10 is a bar graph showing the effect of RIPC on infarction size in mouse hearts. Infarct size expressed as a percentage of area at risk. Black bar represents mice perfused with dialysate from control subjects (n=4) and the grey bar represents mice perfused with dialysate from elite swimmers (n=9). Values are reported as mean ± SEM.

### DETAILED DESCRIPTION OF THE INVENTION

The invention described herein induces an increase in exercise performance without repetitive exercise. It works by inducing natural mechanisms that increase tolerance to inadequate blood flow and reduced oxygen levels in tissues. The stimulus for inducing these natural mechanisms is through a series of brief cessations of blood flow to a limb which induces the state of enhanced exercise performance. This process is known as remote ischemic preconditioning (RIPC). RIPC is a safe process that involves repetitive brief episodes of blood flow cessation to an organ or tissue. While not intending to be bound by any particular theory or mechanism, it is thought that RIPC liberates protective factors in the blood that circulate to tissues around the body, making them more able to tolerate a period of prolonged cessation of blood flow which, in the unprotected state, might otherwise lead to death of those tissues. For example, resistance to tissue death (for example, after restoration of blood flow to the heart muscle during heart attack) can be induced by repetitive brief periods of cessation of blood flow in the arm.

The enhancement of exercise performance is a fundamentally different process than preventing tissue damage resulting from adverse ischemic events such as those discussed above. Heretofore, RIPC has only been shown to be effective in situations where blood flow to tissues has been cut off completely (e.g., such as can occur in a heart attack or stroke). The instant invention contemplates its use in a situation which is quite different. In terms of enhancement of exercise performance, there is no cessation of blood flow to the exercising muscle, and instead the blood flow is maintained or increased. Furthermore, despite the similarity in the induction mechanism, the end effect is quite different. Rather than protecting against cell damage or death, it is rapidly manifest by an improvement in the ability to tolerate exercise in healthy subjects as well as others including those with cardiovascular disease.

The invention therefore relates to the surprising and unexpected discovery that it is possible to improve physical performance by deliberately inducing transient ischemic and reperfusion events, even in the absence of repetitive exercise. In particular, it has also been unexpectedly found that subjecting athletes to such a regimen improves their competitive performance. This is surprising, at least in part, because remote ischemic preconditioning has heretofore been contemplated for use in subjects who were intended to undergo surgical procedures (and would thereby be experiencing ischemia during the surgical procedure) or subjects who due to their present status or medical history (including familial history) were expected to undergo an ischemic event. Thus, RIPC had been previously contemplated for use in treating or preventing an ischemic event or reducing the effects of an ischemic event that was expected to occur. It had not been appreciated, prior to the invention, that RIPC would provide benefit in situations in which blood flow increased or stayed constant, rather than stopped.

The invention, on the other hand, is directed to the use of RIPC to enhance physical performance in subjects. In some embodiments, the subjects are healthy. According to the invention, subjects are not being treated nor is any adverse event being prevented nor is the likelihood that an adverse event will occur being reduced. Rather the invention is directed towards subjects who desire an improvement or enhancement of their level of physical activity or performance. Typically, surgical procedures that induce ischemia and reperfusion events will not be indicated for these subjects (i.e., there is no elective surgery scheduled for such subjects). Broadly speaking, these subjects may be referred to as healthy subjects to the extent that they are not scheduled for any operative procedure that will involve or cause ischemia and reperfusion and in some instances the tissue damage concomitant with such events. The invention therefore in various aspects provides benefit even in the absence of an adverse ischemic event (i.e., an ischemic event other than the transient ischemia induced during RIPC).

The invention is directed even more specifically to athletes, including competitive athletes. Such subjects are under a tremendous pressure to improve performance times and/or other judged end points without the use of prohibited performance enhancing drugs. The RIPC regimen of the invention would satisfy this need as it does not involve administration of any banned substance and more importantly simply takes advantage of inherent processes that operate in the body naturally.

The invention however is not limited solely to athletic subjects and instead can be applied to any subject that will perform a physical activity and in whom an improved performance is desired. The subjects may therefore have average and possibly even below average athletic abilities yet would still be suited for the methods described herein. In some embodiments, the subjects may have poor heart function, heart failure, or other circulatory disturbances that might limit exercise performance.

Such subjects will preferably be humans, although non-human subjects are also contemplated. Such non-human subjects include but again are not limited to any animal used in strenuous competition (e.g., racing) such as horses and dogs.

Thus, in one aspect, the invention provides a method for enhancing physical performance comprising performing a remote ischemic preconditioning regimen on a healthy subject prior to a maximal physical activity.

As used herein, a remote ischemic preconditioning (RIPC) regimen is at least one cycle of an induced transient ischemic event followed by a reperfusion event. Typically, these regimens are performed by restricting blood flow in a limb or a peripheral tissue of the subject (i.e., a "remote location" on the subject) and then removing the blood flow restriction and allowing blood to reperfuse the limb or tissue. A regimen may comprise a single cycle or multiple cycles, including 2, 3, 4, 5, or more cycles. The Examples illustrate performance enhancement using 4 cycles of ischemia and reperfusion.

The blood flow restriction typically takes the form of an applied pressure to the limb or tissue that is above systolic pressure (i.e., supra-systolic pressure). It may be about 5, about 10, about 15, about 20, or more mmHg above (or greater than) systolic pressure. Since systolic pressure will differ between subjects, the absolute pressure needed to induce ischemia may vary between subjects. The blood flow restriction may be accomplished using any method as the invention is not limited in this regard. Typically, it may be accomplished with a standard blood pressure cuff, although a tourniquet is also suitable. Further examples of automated devices for performing RIPC are described below.

The induced ischemic event is transient. That is, it may have a duration of about 1, about 2, about 3, about 4, about 5, or more minutes. Similarly, the reperfusion event may have a duration of about 1, about 2, about 3, about 4, about 5, or more minutes. The Examples demonstrate the effect of 4 cycles of 5 minutes of ischemia followed by 5 minutes of reperfusion on physical performance.

If performed using a limb, the upper limb or lower limb may be used although in some instances the upper limb is preferred. The method may be performed on other remote locations such as but not limited to a foot, a hand, a finger, a toe, or a combination of one or more of any of these.

In some embodiments, the RIPC regimen is performed prior to and typically not during the physical activity. It may be performed within 48 hours, within 24 hours, within 12 hours, within 6 hours, within 4 hours, within 2 hours, within 1 hour, within 30 minutes, within 20 minutes, within 10 minutes, within 5 minutes, or just immediately prior to the physical activity. Each regimen may be performed one or more times, in one day, or per day (daily), or on prescribed days over the course of days, weeks, or months. If two or more regimens are performed, there is no requirement that the regimens be identical with respect to timing, number of cycles, . supra-systolic pressure, and the like. As an example, the length of time of either or both the ischemic event and reperfusion event may vary from one cycle to the next.

In some instances, subject that undergo RIPC prior to maximal physical activity do not demonstrate or experience a change (e.g., an increase) in lactate production as a result of the maximal physical activity.

In addition to their acute effects, the methods of the invention can be used as a long-term training regimen in order to increase muscle activity and performance. Similarly the devices and systems may be used as training aids in order to increase muscle activity and performance.

In some aspects of the invention, the method is intended to improve the performance of a maximal physical activity. As used herein, the term maximal physical activity means an activity in which the subject exerts itself maximally. Exertion levels may be measured in a number of ways known in the art including but not limited to heart rate range, the "talk test", and the Borg rating of perceived exertion (RPE). The degree of activity that yields maximal exertion may vary between certain subjects based on age and physical condition. Nevertheless, methods exist in the art to determine for each subject the level of activity that corresponds to moderate, vigorous or maximal exertion.

The following is a method for determining the level of activity being performed for a given individual using heart rate. Generally, the person's age is subtracted from the hypothetical maximum heart rate of 220. The resulting number is multiplied by a percentage based upon the level of activity being performed. Moderate intensity activity corresponds to about 50-70% of the "age-adjusted" maximum heart rate. Vigorous intensity activity corresponds to 70-85% of the "age-adjusted" maximum heart rate. Maximal activity corresponds to anything higher than 85% of the age-adjusted maximum heart rate.

If the Borg RPE is used, a score of 19 or 20 corresponds to maximal exertion, a score in the range of 15-18 corresponds to vigorous exertion, and a score in the range of 12-14 corresponds to moderate exertion.

In still other embodiments, particularly those which involve subjects with cardiovascular disease, exercise may be limited. In these and other similar situations, an exercise intensity level of NYHA (New York Heart Association) grade 2-4 is contemplated.

Examples of moderate intensity activity include but are not limited to walking briskly (3 miles per hour or faster), water aerobics, bicycling slower than 10 miles per hour, ballroom dancing, tennis (doubles), and general gardening.

Examples of vigorous intensity activity include but are not limited to race walking, jogging or running (e.g., marathon running or racing), swimming laps, tennis (singles), aerobic dancing, bicycling 10 mile per hour or faster, biathlons, triathlons, or other single or multiple activity competitions (e.g., Iron Man competitions), jumping rope, heavy gardening (e.g., continuous digging or hoeing), hiking uphill or with a heavy backpack, and the like.

The activity to be benefited according to the invention may be short (e.g., 60 minutes or less, including 5, 10, 20, 30,40, 50 or more minutes) or it may be long (e.g., more than one hour, including 2, 3, 4, 5, 6 or more hours) in duration.

Physical activity that can also benefit from the methods of the invention includes the activity associated with a rescue operation such as a coast guard rescue operation (e.g., a rescue at sea), activity associated with first-responder activity (e.g., rescuing persons from a burning building), activity associated with hand-to-hand combat military missions, and the like.

Maximal intensity activity could typically be any of the vigorous intensity activities recited herein provided they are performed at the individual subject's maximal ability (i.e., an "all-out" attempt).

It is to be understood that the invention provides methods for improving performance that occurs for any of the foregoing activities since whether a particular activity will require moderate, vigorous or maximum exertion will depend on the individual and their physical ability and condition.

It is also to be understood that the invention contemplates using the RIPC to improve performance for submaximal activities also. The Examples demonstrate that RIPC has the greatest observable effect in competitive swimmers when they are undergoing maximal exertion activity. These subjects are highly trained, and amongst the most physically fit. The invention contemplates performance enhancement for a wider spectrum of subjects, and for a wider spectrum of activity levels including submaximal activity levels (akin to the moderate and vigorous exertion activities discussed herein). It is likely that subjects that are less physically fit than the competitive swimmers of the Examples will benefit from RIPC when performing submaximal activity also.

The methods for measuring performance enhancement will vary based on the particular activity being performed. For example, if the activity is swimming, then the enhancement may be measured by the time to swim a certain distance (e.g., 50 meters, 100 meters, or more). This measurement is shown in the Examples, with a significant difference in 100 meter lap time observed between swimmers who underwent RIPC versus those that underwent sham conditioning. If the activity is running, then the enhancement may be measured by the time to run a certain distance (e.g., 50 meters, 100 meters, 200 meters, 1 mile, a marathon, etc.). Similarly, if the activity is cycling, speed skating, and the like, then the enhancement may be measured by the time to traverse a certain distance. It will be understood that in these examples, the enhancement will be manifested as a decrease in the time taken to perform the activity in question. Other suitable endpoints and readouts will be apparent to those of ordinary skill in the art.

The degree of performance enhancement that can be achieved using the methods provided herein may vary between individuals. The degree of performance enhancement will typically be measured using the difference between the endpoints or readouts achieved following sham conditioning and RIPC. The quotient of that difference and the sham conditioned readout is representative of the improvement achieved.

In some instances, the degree of enhancement may be on the order of 0.5% - 1%, yet still be statistically significant and more importantly competitive or physiologically significant. An example of a 1% enhancement is a decrease of a second for an activity that would take on average 100 seconds to perform in the absence of RIPC. In still other instances, the degree of enhancement may be up to 1.5%, up to 2%, up to 2.5%, up to 3%, up to 3.5%, up to 4%, up to 4.5%, up to 5%, up to 10%, up to 20%, up to 30%, up to 40%, up to 50%, or more.

We have completed a study that has demonstrated this technique to improve exercise capacity in highly trained swimmers. In a randomized, blinded, crossover study, a number of adults underwent RIPC by inflating a blood pressure cuff on the upper arm for 5 minutes to a high enough pressure to occlude blood flow, followed by 5 minutes of restoration of blood flow to the arm after cuff deflation. This process was repeated a further 3 times, consecutively, resulting in four occlusions in total. As a control, on another occasion the same individuals underwent an identical process, but with the blood pressure cuff inflated to just 10 mmHg which does not restrict blood flow to the arm. The swimmer was unaware of the importance of the pressure in the cuff, the observers were blinded to whether the swimmer had RIPC or control procedure, and the order of RIPC and control procedure was randomized (50% RIPC first and then crossing over to control, and 50% control first and then crossing over to RIPC). Consequently, each swimmer performed 2 consecutive 100 meter swims at maximal effort, in a simulated competitive environment, and the swim times compared statistically. There was an average reduction in swim time of approximately 0.7 seconds with RIPC. This represents a statistically significant improvement, that provides competitive and physiological advantage.

This invention is a non-obvious method to improve resistance to exercise-induced fatigue in healthy individuals during sports and activities, and in patients limited by cardiac, circulatory or other medical disorders (e.g., patients with heart failure, angina, peripheral vascular disease, lung disease) that may limit blood flow or muscle power.

RIPC may be performed using any device provided it is capable of inducing transient ischemia and reperfusion, whether manually or automatically.

In one of its simplest forms, the method may be carried out using a sphygmomanometer (i.e., the instrument typically used to measure a subject's blood pressure). The cuff of the sphygmomanometer is placed about a subject's limb (e.g., an arm or leg) and is inflated to a pressure great enough to occlude blood flow through the limb (i.e., a pressure greater than the subject's systolic blood pressure). The cuff is maintained in the inflated state to prevent blood flow through the limb for a specified period of time, referred to herein as the ischemic duration. After the ischemic duration, pressure is released from the cuff to allow reperfusion of blood through the limb for a period of time that is referred herein as the reperfusion duration. The cuff is then re-inflated and the procedure is immediately repeated a number of times.

The method may similarly be carried out using a manual type tourniquet. Devices such as those described in published PCT application WO 83/00995 and in published US application 20060058717 may also be used.

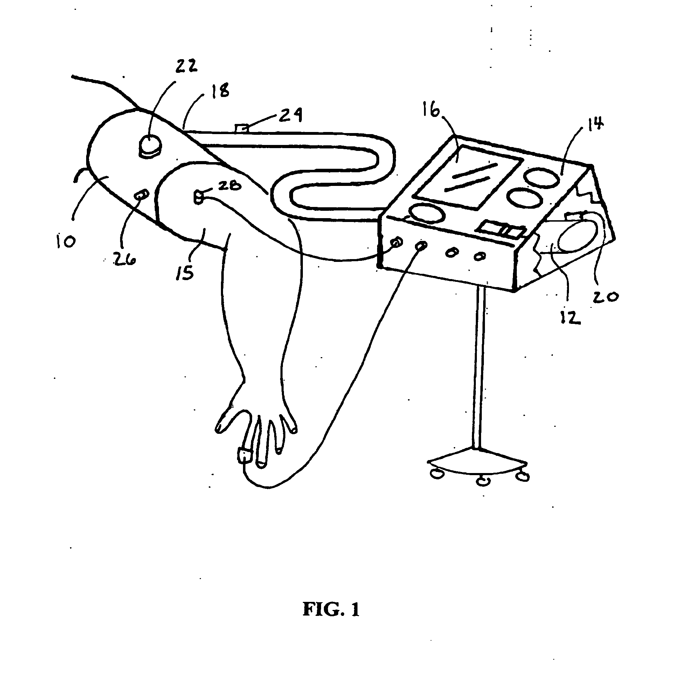

Another system that may be used is described in published US application 20080139949. The advantage of this system is that it can be used independently of a medical practitioner, and that it automatically induces the required RIPC regimen. This system is exemplified in part in FIG. 1, which illustrates a cuff 10, an actuator 12, a controller 14 and a user interface 16. The cuff is configured to be placed about the limb 15 of a subject, such as an arm or leg of the subject. The actuator, when actuated, causes the cuff to retract about the limb to occlude blood flow through the limb. The controller executes a protocol that comprises performing a cycle one or more times. The cycle itself includes actuating the cuff to prevent blood flow, maintaining the cuff in an actuated state for an ischemic duration, releasing the cuff, and maintaining the cuff in a relaxed state to allow reperfusion.

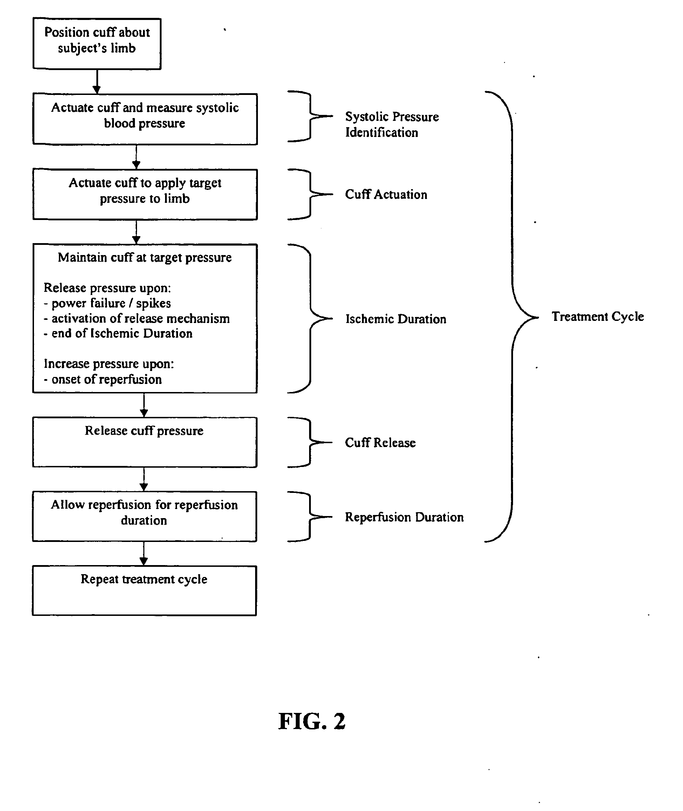

FIG. 2 shows a block diagram that represents a scheme that may be used to perform RIPC. The scheme begins with placement of a cuff about a subject's limb. The system is then activated and the protocol is initiated through the controller. In one embodiment, the system is activated by a medical professional. In another embodiment, the system may be activated by the subject. The cuff contracts to apply an initial pressure, greater than systolic pressure, to the subject's limb. As discussed herein, the initial pressure may be a default value of the system or may be programmed into a particular protocol. The cuff then deflates to identify the subject's systolic pressure. This may be accompanied by monitoring the subject for the onset of Korotkoff sounds or vibrations. Alternatively or additionally, a distal remote sensor (e.g., a device on the fingertip which is sensitive to the presence or absence of flow or maintenance of flow) may be used. Once systolic pressure has been identified, the system initiates the first cycle of the protocol. In some embodiments, systolic pressure may be identified as an initial portion of the protocol. As used herein, the terms protocol and regimen are used interchangeably.

The cycle begins as the cuff contracts to apply a target pressure, greater than the subject's systolic pressure by an amount defined in the protocol, to the subject's limb. This occludes blood flow through the subject's limb. The external pressure against the subject's limb is held for an ischemic duration defined in the protocol. The system monitors the subject during the ischemic duration for pressure release criteria, which may include system power failure, system power spikes, and manual activation of quick release mechanism. The system also monitors the subject during the ischemic duration for any signs of reperfusion through the subject's limb, and accordingly, increases the external pressure applied by the cuff to prevent such reperfusion. Signs of reperfusion can include the onset of Korotkoff sounds or vibrations. After passage of the ischemic duration, the cuff releases pressure from about the subject's limb to allow reperfusion. Reperfusion is allowed for a reperfusion duration defined in the cycle.

The initial cycle typically concludes after the reperfusion duration. At this time, a subsequent cycle may begin as the cuff is actuated to contract about the subject's limb to occlude blood flow through the limb for another ischemic duration.

The cuff illustrated in FIG. 1 is configured to be positioned about the limb of a subject and to contract about the limb when actuated. In one embodiment, the sleeve is wrapped about a subject's upper arm, calf, or thigh and is fastened snuggly in place. Portions of the cuff may include hook and loop type material that can be used to fasten the sleeve in place about the subject's limb. The actuator inflates the cuff such that the limb is constricted to the point of occluding blood flow through the subject's limb.

The illustrated cuff includes an inflatable bladder (not shown) that receives a fluid, such as air, to cause the cuff expand and retract about a subject's limb. The bladder is constructed of an air impermeable material, such as flexible plastic or rubber. A connection port 18 is present at one end of the bladder to allow air to enter the bladder during inflation, or to exit the bladder during deflation. The port may include engagement features to facilitate a connection to the actuator, such as by an air hose. These features may include threads, clips, and the like. Although the illustrated embodiment includes a single bladder positioned within a cuff, it is to be appreciated that other embodiments are also possible. By way of example, according to some embodiments, the fabric sleeve may itself be air impermeable, such that no separate bladder is required. In other embodiments, multiple, separate inflatable bladders may be incorporated into a common sleeve, as aspects of the present invention are not limited in this respect. Alternatively, the cuff may be bladderless. Bladderless cuffs are known in the art. See for example U.S. Patent No. 6036718.

It will be understood that the devices and systems of the invention can comprise one or more cuffs, one or more actuators, and/or one or more controllers. As an example, the device or system may comprise two or more cuffs, one or more actuators (e.g., one actuator for all the cuffs or one actuator per cuff), and a single controller that controls all cuffs and actuators. In devices or systems comprising two or more cuffs, the cuffs may be suitable for use on upper limbs, lower limbs, upper and lower limbs, and/or other remote locations such as hands, feet, fingers and/or toes.

The general size of subjects that undergo RIPC may vary greatly, particularly given the range of species to which the methods may be applied. Given this variance, it may be desirable for some embodiments of cuffs to be adjustable over a wide range to accommodate the variety of subject limb girths that may be expected. According to some embodiments, the cuff comprises an inflatable fabric sleeve having a length greater than three feet, such that a girth of up to three feet may be accommodated. Embodiments of cuffs may include a width as small as two inches, one inch, or even smaller, so as to accommodate the upper arm or leg of a much smaller subject, including a neonatal infant. It is to be appreciated, however, that other embodiments may be configured to encircle a much smaller range of limb sizes, as aspects of the present invention are not limited in this regard.

Various devices may be used as an actuator to constrict the cuff about a subject's limb, or to release the cuff. As illustrated in embodiment of FIG. 1, the actuator includes a pneumatic pump to provide pressurized air to an inflatable cuff through an air hose. The actuator also includes a release valve 20 that, when actuated, opens a passageway between the inflatable cuff and the external environment to allow pressurized air to escape from the cuff, so that the cuff loosens about the subject's limb.

The air pump can comprise any device capable of delivering compressed air. According to some embodiments, the air pump includes a piston compressor, although other types of pumps, like centrifugal pumps and scroll compressor may also be used. The pump may be configured to provide air flow at a rate of between 0.1 to 20 cubic feet per minute, with a head pressure of up to 50 psi, according to some embodiments. However, other flow rates and/or pressures are possible, as aspects of the invention are not limited in this respect.

As discussed above, the actuator may also include a release mechanism to release a cuff from about the subject's limb. In the illustrated embodiment, the release comprises a release valve 20 that is positioned within the controller housing. The release valve, as shown, may be a solenoid that moves rapidly between fully closed and fully open positions to rapidly release air from the cuff and, in turn, to rapidly release the cuff from a subject. According to some embodiments, the same release valve or another release valve may also be actuated to open slowly, such as to adjust the pressure of the cuff or to allow a more controlled release of pressure such as may be required when the subject's blood pressure is measured.

Embodiments of the system may include safety features to allow rapid release of the cuff from a subject's limb. Moreover, some of these embodiments may be readily activated by a subject, such as when the subject feels discomfort. In one embodiment, the safety release 22 includes a large button positioned on or near the cuff. In this regard, the safety release is within reach of the subject. In other embodiments, the safety release may comprise a separate actuator, such as one that may be held in the free hand of the subject. Activating the safety release may cause the release valve of a pneumatic cuff to open, thereby allowing rapid removal of air from the cuff.

The system may also include a continually operating, cuff release mechanism. By way of example, a slow release valve may be incorporated into a pneumatic cuff to provide for a continual, slow release of pressurized air from the cuff. The continual slow release mechanism may provide for the safe release of a subject's limb, even in the face of power failures or other events that may prevent redundant safety features from operating properly. Similar type mechanism may be incorporated into embodiments that do not utilize a pneumatically inflatable cuff, as continual slow release mechanisms are not limited to pneumatic cuffs.

Embodiments of the system include a controller that receives information from a protocol and any other sensors in the system to, in turn, control the actuator to perform RIPC. The controller and protocol combination may be implemented in any of numerous ways. For example, in one embodiment the controller and protocol combination may be implemented using hardware, software or a combination thereof. When implemented in software, the software code can be executed on any suitable processor or collection of processors, whether provided in a single computer or distributed among multiple computers. It should be appreciated that any component or collection of components that perform the functions described herein can be generically considered as one or more controllers that control the functions discussed herein. The one or more controllers can be implemented in numerous ways, such as with dedicated hardware, or with general purpose hardware (e.g., one or more processors) that is programmed using microcode or software to perform the functions recited above. The one or more controllers may be included in one or more host computers, one or more storage systems, or any other type of computer that may include one or more storage devices coupled to the one or more controllers. In one embodiment, the controller includes a communication link to communicate wirelessly, or via electrical or optical cable, to a remote location.

In this respect, it should be appreciated that one implementation of the embodiments of the present invention comprises at least one computer-readable medium (e.g., a computer memory, a floppy disk, a compact disk, a tape, etc.) encoded with a protocol in the form of a computer program (i.e., a plurality of instructions), which, when executed by the controller, performs the herein-discussed functions of the embodiments of the present invention. The computer-readable medium can be transportable such that the protocol stored thereon can be loaded onto any computer system resource to implement the aspects of the present invention discussed herein. In addition, it should be appreciated that the reference to a protocol or controller which, when executed, performs the herein-discussed functions, is not limited to an application program running on a host computer. Rather, the term protocol is used herein in a generic sense to reference any type of computer code (e.g., software or microcode) that can be employed to program a processor to implement the herein-discussed aspects of the present invention.

The system may also comprise one or more sensors 26 that receive information from the subject and/or portions of the system itself. Such sensors may receive information regarding blood flow in any portion of the subject, including the limb that is being treated. These sensors may also receive information regarding other operating parameters of the system, such as air pressure within a pneumatic cuff, direct readings of pressure applied by cuff, or tension within portions of a tension band.

Pneumatic cuffs may include a sensor to measure pressure within the cuff. Cuff pressure is often directly indicative of the pressure that exists within a blood vessel of the limb beneath the cuff. The controller of a system is often programmed to target a particular cuff pressure that is to be maintained during the ischemic duration of a cycle, as is discussed herein. In embodiments that include a pneumatic cuff, the pressure sensor may be positioned anywhere within the pressurized space of the cuff, the air hose, or even within the actuator itself. Pressure sensors may also be positioned on an inner surface of the cuff to directly measure the pressure between the cuff and an outer surface of the subject's limb. In use, the cuff may be oriented such that the pressure sensor is positioned directly above the subject's artery, so as to provide a more direct measurement of pressure at a blood vessel of interest.

In one embodiment, systems may also include one or more vibration and/or ultrasonic sensors 28 to identify Korotkoff sounds. Korotkoff sounds are generally understood to be present when pressures between systolic and diastolic are externally applied to the artery of a subject. Systolic pressure is associated with a pressure value that completely occludes blood flow through a subject's blood vessels, and in this regard, may be used by the system as feedback to identify when pressure in the system is low enough to allow blood flow, or high enough to occlude blood flow.

One or more sensors may be included to confirm the cessation of blood flow or reperfusion in the limb that receives the cuff. For instance, in some embodiments, a pulse oximeter 30 may be positioned on a distal portion of the limb that receives the cuff, such as on a finger or toe of the limb. The pulse oximeter can provide information regarding blood pulsing through the subject's blood vessels and the percentage of haemoglobin that is saturated with oxygen. The pulse oximeter will detect an absence of pulses when blood flow though a limb is not occurring to confirm the occlusion of blood flow. Moreover, the pulse oximeter may also detect the percentage of haemoglobin saturated with oxygen, which will drop as blood flow through the limb ceases. It is to be appreciated that other sensors may also be used to confirm the cessation of blood flow, such as a photoplethysmographic transducer, an ultrasonic flow transducer, a temperature transducer, an infrared detector, and a near infrared transducer, as aspects of the invention are not limited in this respect.

As mentioned above, the system includes a protocol that, through the controller, directs the operation of the system. Embodiments of the protocol include a cycle that comprises cuff actuation, an ischemic duration, cuff release, and a reperfusion duration. In many embodiments of protocols, the cycle may be repeated multiple times. Additionally, some embodiments of the protocol include systolic pressure identification.

The cuff actuation portion of the cycle comprises contracting the cuff about the limb of a subject to occlude blood flow through the limb. Contraction of the cuff is accomplished by the controller reading instructions from the protocol, such as a target set point for cuff pressure, and then by the initiating the controller to bring the cuff to the target set point. Attainment of the target set point may be sensed trough any of the herein described sensors and techniques.

During the ischemic phase of the cycle, pressure is maintained about the subject's limb to prevent reperfusion of blood flow through the limb. The length of the ischemic phase, termed the ischemic duration, is typically defined by a doctor, or other medical professional, and is programmed into the protocol. Ischemic duration may be as short as a few seconds, or as long as 20 minutes, or even longer, as aspects of the invention are not limited in this regard. In some embodiments, the ischemic duration varies from cycle to cycle during the same protocol, although in other embodiments, the ischemic duration remains constant.

The controller acts to maintain pressure, applied by the cuff, at a set point above the subject's systolic pressure. Embodiments of the cuff may relax relative to the subject's limb over time, thereby reducing pressure and eventually allowing reperfusion. This may be caused by various factors, including relaxation of muscles in the subject's limb, stretching of the cuff about the limb, air leaks (intentional or unintentional), and the like. To this end, a sensor may provide pressure readings as feedback to the controller. The controller can measure any difference between the set point and the actual pressure reading and can provide any necessary commands to the actuator to compensate for errors.

Various approaches may be used to define an appropriate set point for the controller during the ischemic duration. According to one embodiment, the set point is manually entered into the protocol by the doctor (or other medical professional). Alternately, the doctor may select a set point in terms of the subject's systolic blood pressure. In one embodiment, the set point may be selected as a fixed pressure amount over the subject's systolic blood pressure, such as 5 mm Hg, 10 mm Hg, 15 mm Hg, 20 mm Hg, 25 mm Hg, 30 mm Hg, or any other fixed amount above systolic pressure of the subject. In other embodiments, the set point may be defined as a percentage of the subject's systolic blood pressure, such as 102% of systolic, 105%, 110%, 115%, and other percentages, as aspects of the invention are not limited in this respect. The point above systolic pressure may be set by the medical professional and may be dependent upon several factors including, but not limited to the size of the subject, the size of the subject's limb, the subject's blood pressure, confirmation of blood flow cessation, and the like.

The protocol, according to some embodiments, includes phases to identify the subject's systolic blood pressure. The cuff may be allowed to loosen about the subject's limb, from a point believed to be above systolic pressure, in a systematic manner while sensors are monitoring the limb for the onset of Korotkoff sounds or vibrations. Once the systolic pressure is identified, the protocol may continue in the normal course.

Identification of systolic pressure may optionally occur at any time during a protocol, or not at all. According to some embodiments, each cycle begins with the identification of the subject's systolic blood pressure. In other embodiments, systolic pressure may be identified only once during an initial portion of the protocol. In still other embodiments, systolic pressure may be identified as the cuff is released during the cuff release portion of each cycle. Still, as discuss herein, systolic pressure may not be identified at all during a protocol, as aspects of the invention are not limited in this regard.

The system can be configured to adjust the pressure set point during the ischemic duration. As discussed herein, the system may include sensors that detect the onset of reperfusion. As an example, this may be accomplished by detecting the presence of Korotkoff sounds or vibrations. The presence of Korotkoff sounds during an ischemic duration can indicate that either cuff pressure has fallen below systolic or that systolic pressure has risen above the set point that was previously above systolic pressure. Other devices may additionally or alternatively be used including for example devices on digits that detect the presence or absence of flow. In such a situation, the controller may adjust the set point based on the newly identified systolic pressure and/or other information and in this regard, can identify and prevent unwanted reperfusion that might otherwise occur.

The cuff release portion of a cycle occurs at the end of the ischemic duration and includes release of the cuff to a point below diastolic pressure. According to some embodiments, cuff release comprises releasing the pressure or tension of the cuff. In embodiments that utilize a pneumatic cuff, this may simply be associated with moving an air release valve to the fully open position to allow a rapid reduction in cuff pressure and a corresponding rapid relaxation of the cuff about the subject's limb. However, it is to be appreciated, that in other embodiments, that cuff relaxation may occur in a slower, more controlled manner, as aspects of the invention are not limited in this respect. Additionally, as discussed herein, the cuff release may be accompanied by monitoring for the onset of Korotkoff sounds or vibrations to identify or confirm the systolic pressure of the subject.

The reperfusion duration follows the cuff release in embodiments of the cycle. Reperfusion through the limb is allowed for a period of time termed the reperfusion duration. Much like the ischemic duration, reperfusion may be allowed for varied lengths of time, as short as a five seconds, one minute or more, and as long as 20 minutes, or even longer. The reperfusion duration may remain constant from cycle to cycle during a common protocol, or may vary between each cycle, as aspects of the invention are not limited in this respect.

The protocol may comprise any number of cycles. As discussed herein, a common cycle may simply be repeated a plurality of times, such as two, three, four, or more times, to complete a protocol. Alternately, the cycles of a protocol may be programmed with different parameters, such as different ischemic durations, reperfusion durations, pressure set points during the ischemic duration, and the like.

In some embodiments, the system may include a data logging feature that records the system parameters, such as cuff pressure or tension, during all phases of a protocol. Date of time of operation may also be recorded. Other features, such as personal information to identify the subject, may also be recorded by the system.

Embodiments of the system may incorporate various features to inform the subject or medical professional about the progress of the protocol. Audible or visual indicators may accompany any of the phases of the protocol. By way of example, a clock may show either the amount of time that has elapsed or that remains for a given portion of the protocol or the entire protocol. Embodiments may also include other features to keep the subject and/or medical professional informed, as aspects of the invention are not limited in this regard.

According to some embodiments, the system includes features to prevent tampering or accidental reprogramming by a subject. By way of example, in some embodiments, the reprogrammable features may only be accessed after entering a code. This can prevent a subject from mistakenly reprogramming the protocol or otherwise interfering with the operation of the system. It is to be appreciated that other devices may also be used to prevent accidental reprogramming, such as electronic keys, mechanical locks and the like.

The system may be configured for use is a variety of environments. By way of example, the system may be mounted on a portable stand with casters to facilitate easy movement. The stand may position the controller, user interface, and connections to the cuff at a convenient height for the subject. In other embodiments, the system is configured for portable use. In such embodiments, the system may be configured for ready placement into a suitcase for easy transport.

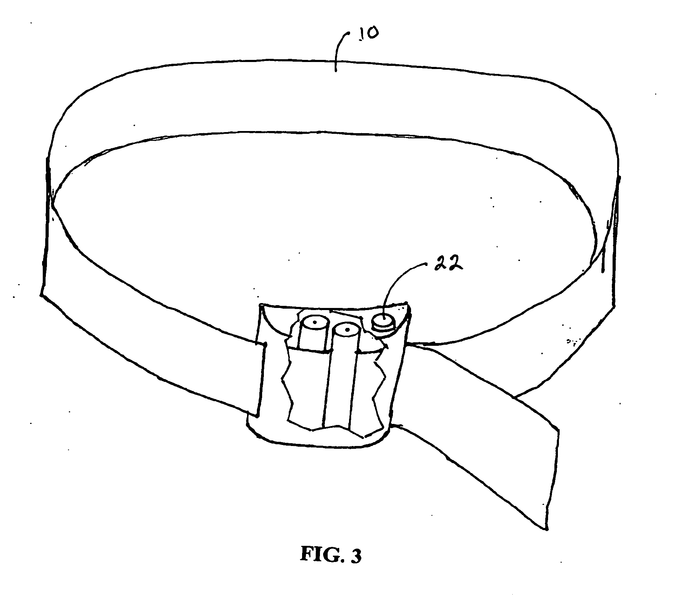

The system is also not limited to components illustrated in the embodiment of FIG. 1. By way of example, according to other embodiments, like that illustrated in FIG. 3, cuffs may be configured to constrict a subject's limb through alternative mechanisms. In the illustrated embodiment, the cuff is configured as a band having a ratcheting mechanism positioned at one end. In use, the band is wrapped about the limb of a subject with the free end of the band passing through the ratcheting mechanism. In such an embodiment, the actuator may comprise a mechanism that pulls the free end of the band further through the ratcheting mechanism to retract the cuff about the limb, or that frees the ratcheting mechanism to release the band to, in turn, release the band from the limb. Still other mechanisms, such as tourniquet mechanisms, are possible, as aspects of the invention are not limited in this respect.

As described above with reference to FIG. 3, some embodiments may have a cuff that comprises a band that does not inflate, but rather is tightened about a subject's limb by another mechanism. In such embodiments, the actuator may comprise a tensioning mechanism configured to move one end of the band relative to other portions of the band so as to place the band in tension. As shown, the mechanism can include opposed rollers held in close proximity to one another within a housing. The housing includes a slot for receiving a free end of the band and a fixation point for fixed attachment to the opposite end of the band. The free end of the band is passed into the slot and between the rollers. The rollers may be mechanically actuated to rotate relative to one another, such as by an electric motor, to pull the free end through the housing and thus tighten the band around a subject's limb.

The tensioning mechanism may include opposed rollers mounted on a ratcheting, free wheel mechanism. The freewheel mechanism allows the band to be pulled through the slot in one direction with minimal resistance so that the band may be pulled rapidly to a snug position about a subject's limb. The free wheel mechanism also prevents the band from moving through the slot in the loosening direction, unless the mechanism is released or the opposed rollers are actuated. It is to be appreciated that not all embodiments will include a free wheel mechanism, as aspects of the invention are not limited in this regard.

The opposed rollers rotate in either direction to tighten and loosen the band during use. When required, the rollers may rapidly rotate until the band achieves a particular tension. The rollers may further be actuated to make minor adjustments to the tension in the band during use. When the cuff is to be released from the subject's limb, a ratcheting mechanism or clutch may be released such that the opposed rollers are allowed to move freely, thus rapidly releasing tension.

One aspect of the invention therefore provides a system or device for remote ischemic preconditioning, the system comprising a cuff configured to retract about a remote location of a subject; an actuator connected to the cuff and that, when actuated, causes the cuff to contract about the remote location of the subject to reduce blood flow there through; and a controller that controls the actuator to operate according to a treatment protocol that includes a plurality of sequentially actuated treatment cycles, each treatment cycle comprising: cuff actuation, during which the actuator contracts the cuff about the remote location of the subject to a pressure above systolic pressure to occlude blood flow through the remote location; an ischemic duration, during which the actuator maintains the cuff contracted about the remote location at a set point above systolic pressure to occlude blood flow through the remote location, the ischemic duration lasting for at least about a minute; cuff release, during which the actuator releases the cuff to allow blood flow through the remote location; and a reperfusion duration, during which the cuff is maintained in an at least partially relaxed state to allow blood flow through the remote location, the reperfusion duration lasting for at least one minute. The remote location may be a limb, although it is not so limited.

The device may be particularly suited for use in an athletic environment. As an example, the cuff may be configured in a manner to be secured to a remote location such as an arm or a leg. As an example, it may be associated with an elasticized housing or it may be elasticized itself (provided that it does not impact the ischemic and reperfusion events contemplated by the invention). Alternatively or additionally, it may be attached to or incorporated into clothes including socks, shoes, running apparel (running suits, running jackets, running pants), swimming apparel (e.g., swim suits, including fastskin suits, etc.), and the like, in whole or in part. In the case of a full body suit, the device may be in the arm(s) and/or in the leg(s) part of the suit. The suit may be so configured as to provide an external cord or outlet to or into which a power cord may be connected or to which other elements of the device may be connected (e.g. the controller, the power source, and the like). The suit may alternatively configured such that the device can be threaded between two materials or between two layers comprised in the suit. In still other embodiments, a shoulder harness and/or a belt may be associated with the device in order to provide support. The device may comprise a waterproof housing, or it may be otherwise designed to be waterproof, or it may have waterproof elements. The device may comprise a housing that protects it from bodily fluids such as sweat (e.g., it may be "sweat-proof"). In these and other embodiments, the device may comprise a plastic or other water-resistant housing.

In the event the device is used for animals such as racing breeds (e.g., dogs, horses, etc.), it may be suitably configured for such use. As an example, the device may be provided or encased in the covers (e.g., capes) used to keep animals (e.g., horses) warm prior to competition.

As used herein, a garment is any form of clothing or apparel. The garment may be clothing or apparel that is worn during physical activity or during a warm-up period prior to physical activity. The device (or system) may be provided on an inner layer or surface of the garment so that it contacts the subject. The device (or system) may be provided between layers of the garment so that it does not contact the subject. When provided in association with a garment, the device (or system) may comprise the cuff and optionally the actuator. In some instances, the device will not comprise a power source (e.g., batteries and/or cords) and/or the device will not comprise a controller. The cuff-comprising device may be connected to an actuator and/or a controller and/or a power source when a remote ischemic preconditioning regimen is to be performed. The incorporation of a cuff-comprising device (without an actuator and/or a controller and/or a power source) will therefore limit the mass (or weight) added to the garment, thereby allowing the garment to be worn throughout training and performance time periods. The cuff may be disposable, in some instances. The garment may be provided together with a portable, self-contained (stand alone) actuator and/or a portable, self-contained (stand alone) controller and/or a portable, self-containing (stand alone) power source or supply. It will therefore be understood that one or more elements of the device (e.g., the cuff) may be associated with a garment (and therefore be referred to as integral to or incorporated within the garment), and/or that one or more elements of the device (e.g., the power source or supply) may be physically separate from the garment. The invention contemplates a kit that comprises the garment and any physically separate elements of the device, including the actuator, the controller, and/or the power source or supply.

The invention further contemplates operation of the device through direct connections or wirelessly. Wireless operation may comprise wireless control of the device. As an example, the controller may be physically separate from the cuff and the actuator but may be in wireless contact with one or both elements. This configuration allows the cuff and/or actuator to be controlled at a distance, and may reduce the overall weight of the device. Wireless controllers include mobile devices including smart phones and other wireless hand-held devices that can be programmed and/or can upload computer applications that will control the operation of the actuator and/or cuff. The wireless controller may also direct the power source or supply to turn off and/or on. Non-limiting examples of commercially available wireless controllers are an iPhone™, an iPod™, an iPad™, a Blackberry™, and the like. As will be clear, such wireless devices will allow for remote control of the cuff and/or actuator. In such instances, the cuff and/or the actuator may further comprise an override mechanism that allows the subject wearing the garment (or someone in the vicinity) to override a remote instruction.

Aspects of the invention are not limited to the embodiments of cuffs illustrated herein.

### EXAMPLES

### Example 1: Exercise performance in competitive athletes.

Exercise performance in elite athletes, and swimmers in particular, is thought to be limited by skeletal, cardiac and respiratory muscle fatigue. Here, we show that deliberate induction of repetitive cycles of ischemia and reperfusion can enhance the performance of such elite athletes.

### Summary

11 national level swimmers, 13 to 18 years of age, were randomised to real or sham preconditioning. The preconditioning protocol consisted of 4 cycles of upper limb ischemia (5 minutes - standard blood pressure cuff inflated to supra-systolic pressure) and reperfusion (5 minutes). Sham preconditioning intervention consisted of the same protocol, but with the cuff inflated to a pressure of 10 mmHg. Every subject crossed-over the following week. In study 1, subjects performed two standardised submaximal incremental swimming performance tests with measurement of swimming velocity, blood lactate, and heart rate between each increment. Study 2 (n=8) examined the effects of RIPC during 2 consecutive competitive swims of 100 meters using the swimmer's preferred stroke, in order to assess the effects on maximal exercise performance. All measurements were recorded by observers blinded to the treatment protocol.

There was no effect of RIPC on incremental submaximal exercise performance in study 1. Critical velocity (the relation between swimming velocity and the heart rate) was 1.36 ± 0.12 after sham vs. 1.35 ± 0.12 m/s after RIPC, p=.0.50. Similarly, lactate (13.6 ± 2.4 vs. 12.9 ± 2.7 mmol/L, p=0.22) and maximal heart rate (187.0 ± 9.9 vs. 188.0 ± 10.0, BPM, p=0.60) were also unchanged. However, in study 2 the increase in stroke number associated with RIPC was not significant (28.6 ± 8.8 (sham) vs. 29.6 ± 8.3 str/50 m, p=0.17) but a significantly improved swim time was observed (64.9 ± 3.9 (sham) vs. 64.0 ± 3.7s, p<0.05). This improvement was not at the expense of increased lactate production (p=0.46). Using linear regression models adjusted for repeated measures and order of treatment assignment, there was a 0.93 ± 0.35s reduction in competitive swim time (p=0.009) with RIPC.

### Introduction

Ischemia is caused by an abrupt discontinuation of blood flow and therefore of oxygen supply to a specific organ or tissue. Ischemic-reperfusion injury has been implicated in a multitude of diseases and can be caused by different mechanisms the most common being occlusive blood clots, poor systemic perfusion, and vascular injury. For example, ischemic myocardial disease, which is the leading cause of mortality in adults (ref. 1), is caused by a blood clot obstructing an injured coronary artery or a bypassed vessel. Ischemic preconditioning is the most potent endogenous mechanism that has been demonstrated to protect tissue against ischemia-reperfusion injury. The protective effect of this *local* ischemic preconditioning results from short, non-lethal ischemic episodes to the target tissue (ref. 2, 3). However, preconditioning can be difficult to apply therapeutically because it requires short term ischemia in the target organ, thus potentially resulting in dysfunction in the very organ that requires protection.

Remote preconditioning (RIPC) is a more clinically relevant stimulus. This concept was initially described in different regions of animal hearts (ref. 4). We recently demonstrated that this concept can be expanded to humans, showing that four 5 minute episodes of ischemia to limb skeletal muscles, induced by inflating a standard blood pressure cuff higher than systolic blood pressure, protects the heart and lungs against ischemia-reperfusion injury in children undergoing cardiac surgery using cardiopulmonary bypass (ref. 5).

The invention relates to the use of RIPC to enhance exercise performance. Swimming, in particular, represents a unique physiological challenge. Swimmers must contend with restrictions placed on their breathing frequency during intense exercise, resulting in a unique interaction between muscle physiology, technique, and ventilation. Exercise hyperpnoea is limited during high intensity swimming (ref. 6) because turning or lifting the head to breathe may jeopardize execution of proper stroke technique (ref. 7). During high intensity swimming, breath holding can result in significant decreases in the arterial partial pressure of oxygen (PaO₂), decreased blood pH (increased [H⁺]) but unchanged arterial partial pressure of carbon dioxide (PaCO₂) relative to non frequency-controlled breathing (PaO₂ unchanged, pH unchanged, PaCO₂ decreased) (ref. 8). Swimming requires that the athlete sustain a high rate of energy expenditure and the suspension of breathing (through breath holding) for approximately 20 to 30 percent of a race (ref. 9). Given these limitations and their physiological consequences, it is likely that exercise-induced arterial hypoxemia (EIAH) would occur during competitive swimming. This EIAH may be a significant contributor to the development of fatigue in skeletal, respiratory and cardiac muscles (ref. 10) responsible for the physiological limitation in maximal swimming exercise.

We demonstrate here that remote preconditioning prior to exercise renders tissues more resistant to the adverse metabolic effects of extreme exercise, in much the way it modifies tissue responses to ischemia, and thus that swimming performance is modifiable by remote preconditioning.

### Methods

The Hospital for Sick Children Research Ethics Board approved the protocol which was registered prior to study initiation (Identifier: NCT00761566). Subjects were selected from competitive swimming teams from the city of Toronto, Canada. Healthy male or female swimmers between 13 and 18 years of age who had previously achieved a swimming performance time within national championship qualification standards were included in the study. Subjects with diabetes mellitus, a recent illness, recent surgery or any medical intervention in the 48 hours prior to any of the study days were excluded. Informed consent was obtained before enrollment in the study.

The randomization list was computer generated. The randomization codes were sealed in opaque envelopes. The athletes were assigned envelopes after being included in the study. Subjects were randomised to receive either four 5 minute cycles of upper limb ischemia interspaced with 5 minutes of reperfusion or a sham procedure. Ischemia was achieved by using a blood-pressure cuff inflated to a pressure of 15 mmHg greater than systolic arterial pressure whereas, using the same cycling protocol, the blood pressure cuff was only inflated to 10 mmHg for the sham procedure. The 'reperfusion' period consisted of 5 minutes after full cuff deflation. On the subsequent study date, separated by one week from the previous one, the subjects were submitted to the intervention they had not received. All study investigators and participants were blinded to treatment assignment for the duration of the study. The group allocation was not revealed to the investigators until the end of the statistical analysis and the athletes were not told which inflation could be beneficial to their swimming performance. Before the different swimming performance tests, the swimmers swam their normal warm-up.

The submaximal exercise swimming protocol used in this Example has been previously validated (ref. 7, 11). The test was conducted in a long course pool (i.e. 50 meters in length). Before the swimming test, participant's weight and height was measured (ref. 12). Each submaximal swim test consisted of a set of 7 x 200 meters swims on a pace time of 6 minutes beginning from a push start. This meant that the length of the rest period before the subsequent effort was determined by the amount of time taken to complete the 200 meters. The researcher calculated the required speed for each 200 meters swim prior to the test and the participants were informed of these target speeds before the test began. Each target speed was based on a fixed percentage of the participant's best time. For example, the first 200 meters were swum at a speed that would result in a time equal to the individual's best time + 35 seconds. Thereafter, each subsequent 200 meters were completed 5 seconds faster than the preceding one. The final (7^{th}) 200 meters swim is an "all out" maximal effort performance. Time, heart rate, stroke rate and blood lactate were measured and recorded for each swimming increment. The swimmers were asked to swim the performance test in their best stroke style (e.g. freestyle, backstroke, breaststroke, fly, individual medley).

The maximal swimming performance test was also completed in a long course pool. The swimmers were required to swim 100 meters using their best stroke style at 100% effort. Blood lactates were measured before and after the test. Time and stroke rate were also measured.

The primary endpoint of this study was an improvement in the critical velocity, defined as the relation between the mean heart rate and swimming velocity, of preconditioned subjects during submaximal exercise testing. The primary end point for the maximal exercise test was the swim time (calculated as the total time in seconds required to swim 100 meters). Secondary endpoints were reduction in peak blood lactate level and improvement of stroke rate.

### Statistical Analysis

Data are presented as means with standard deviations, medians with minimum and maximum values and frequencies as appropriate. Unadjusted differences between groups were assessed using Fisher's exact chi-square and paired t-tests. Linear regression models adjusted for repeated measures and order of treatment assignment were created to evaluate differences between groups beyond randomization. Further adjustments in regression models for gender, style, age at test, height, weight and international ranking did not change the results. Statistical analysis was limited to patients who completed both study interventions. All statistical analyses were performed using SAS statistical software v9.1 (The SAS Institute, Cary, NC).

### Results

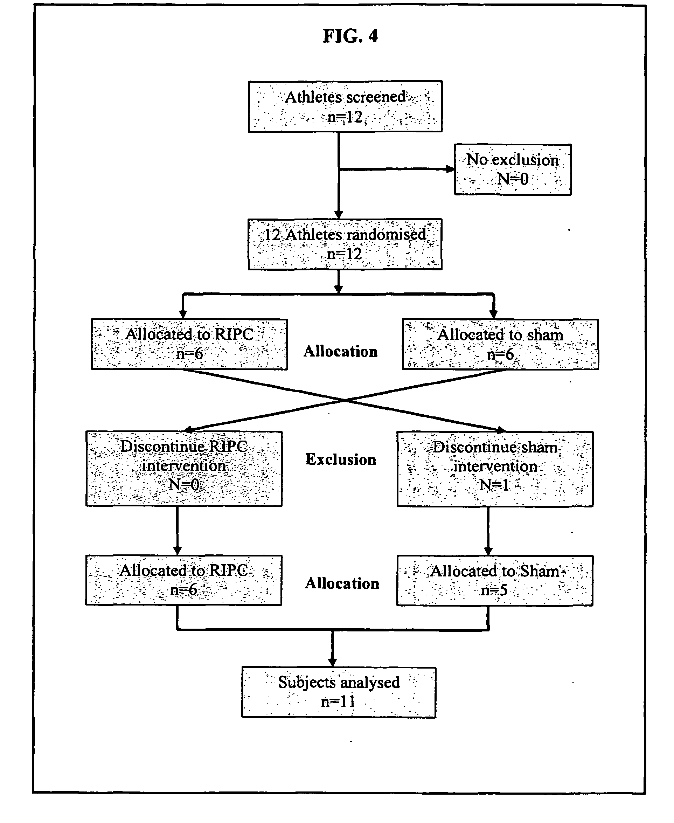

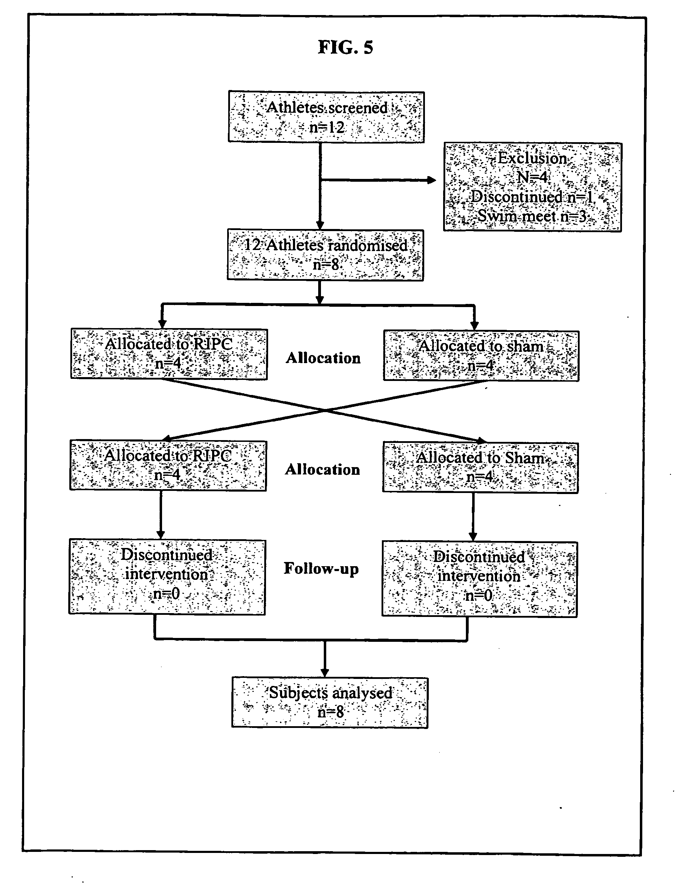

A total of 12 athletes were eligible for randomisation. The submaximal exercise test was completed first (FIG. 4) and 11 subjects completed both interventions. Subsequently, 8 subjects received both interventions for the second part of the study, the maximal exercise performance test (FIG. 5). One athlete withdrew from the study on the second testing day due to illness. Three athletes were unable to participate in the maximal performance testing because of a conflict with their competition schedule. Table 1 provides the characteristics of the elite swimmers included in the study. There were no protocol deviations.

This small study did not demonstrate an observable effect of RIPC on submaximal exercise performance in elite athletes. In models both adjusted and unadjusted for order of treatment assignment we were not able to find any significant effects of RIPC on any of the indicators of exercise performance. Table 2 shows the effect of RIPC on all the indicators of submaximal performance. There was no observable effect on critical velocity defined as the relation between swimming velocity and heart rate. Maximal heart rate and the heart rate slope appeared unchanged by the preconditioning protocol. Finally, the velocity obtained at a lactate concentration of 4 mmol/L was also seemingly unaffected. Table 3 shows the effect of RIPC on the same exercise submaximal performance indicator using linear regression models adjusted for repeated measures and order of treatment assignment.

In elite swimmers, RIPC was associated with an improvement in maximal performance without a change in lactate production. Table 4 shows the effect of RIPC on the indicators of maximal performance. RIPC was associated with a significant improvement in competitive swim time for 100 meters and a tendency towards increased stroke number. Moreover, this improvement in swim time was not achieved at the expense of increased lactate production. Finally, using linear regression models adjusted for repeated measures and order of treatment assignment, there was a 0.93 ± 0.35s reduction in competitive swim time (p=0.009) (Table 5).

No adverse events were noted for either the sham or the RIPC interventions.

### Discussion

In this study, the first to assess the effects of preconditioning on exercise performance, RIPC was associated with an improved maximal performance in the elite swimmers. RIPC was associated with a reduction in the time needed to swim 100 meters at 100% effort by 0.93 (±0.35, p=0.009) seconds.

RIPC is a phenomenon that is known to protect tissue against ischemia and reperfusion injury, usually as a result of cessation of blood flow to a tissue bed, such as occurs during cardiac surgery, or myocardial infarction. Although exercise is associated with hyperemia, we hypothesized and here demonstrated that RIPC can modify the response of exercising muscles. While not intending to be bound by any particular theory or mechanism, it is hypothesized that RIPC allows for faster uptake of Acetyl-CoA (a breakdown product of glycolysis) by mitochondria, thus maintaining lactate accumulation at a metabolically acceptable level and contributing aerobically-generated ATP for exercise.

Regardless of the mechanism, the 0.93 seconds reduction in time is statistically significant, and also is of major physiologic and competitive importance to the swimmers, as it represents a 1.45% improvement in swim time. It has previously been suggested that an improvement of 0.4% in competition performance is a 'competitively significant' change (ref. 16). The most important factor that has been shown heretofore to improve swimming performance is training. However, other adjuncts such as respiratory muscle training (ref. 11) and fastskin suits (ref. 17) have also been proven to improve swimming performance. For example, athletes swimming with the full body fastskin suit had an improvement in performance by an average by 2%, leading to their widespread use in competition. Our data show that RIPC is a useful and, given its 'natural' mechanism, legal adjunct to performance enhancement.

### Example 2: Updated study on competitive athletes.

### Summary

National level swimmers, 13 to 27 years of age, were randomized to RIPC (4 cycles of 5 minutes arm ischemia/5 minutes reperfusion) or sham, with crossover. In study 1, subjects (n=16) performed two incremental submaximal swimming tests with measurement of swimming velocity, blood lactate, and heart rate. For study 2, subjects (n=18) performed two maximal competitive swims. To examine possible mechanisms, blood samples taken before and after RIPC were dialysed and used to perfuse mouse hearts (n=10) in a Langendorff preparation. Infarct sizes were compared to dialysate obtained from non athletic controls pre and post RIPC. RIPC released a protective factor into the blood stream that reduced infarct size in mice (p<0.05 for control subjects and swimmers). There was no effect of RIPC on submaximal exercise performance. However, RIPC was associated with a mean improvement of maximal swim time for 100 meters of 0.7 second (p=0·04), an improvement in swim time relative to personal best time (p=0·02), and a significant improvement in average international swimming federation points (p=0·01).

RIPC improves competitive performance in elite swimmers. This technique is applicable to other sports and clinical syndromes in which exercise tolerance is limited.

### Methods

A double-blind cross-over randomized control trial was performed. The Hospital for Sick Children Research Ethics Board approved the protocol which was registered prior to study initiation (Identifier: NCT00761566, registered November 2008). Subjects were selected from Canadian competitive swimming teams at both the national and international level. Healthy male or female swimmers between 13 and 27 years of age who had previously achieved a swimming performance time within national championship qualification standards were included in the study. The swimmers' best performances were evaluated using an international point score system (ref. 27) recognized by the international swimming federation (FINA) which permits the comparison of performance by male and female swimmers in any of the different swimming events (freestyle, backstroke, breaststroke, butterfly, and individual medley). This system ascribes a point score to each swim scaled to 1000 points (a score of 1000 points is equal to the mean of the eight fastest times in the history for that event). Subjects with scores above 700 were included in the study. Subjects with diabetes mellitus, a recent illness, recent surgery or any medical intervention in the 48 hours prior to any of the study days were excluded. Informed consent was obtained before enrollment in the study. On a separate occasion, we performed an experimental study in a sub-group of control subjects and swimmers to assess the release of humoral preconditioning factors during RIPC. Blood samples were obtained before and after RIPC, using an identical RIPC protocol, and prepared for in-vitro validation using our previously described Langendorff method (ref. 21).

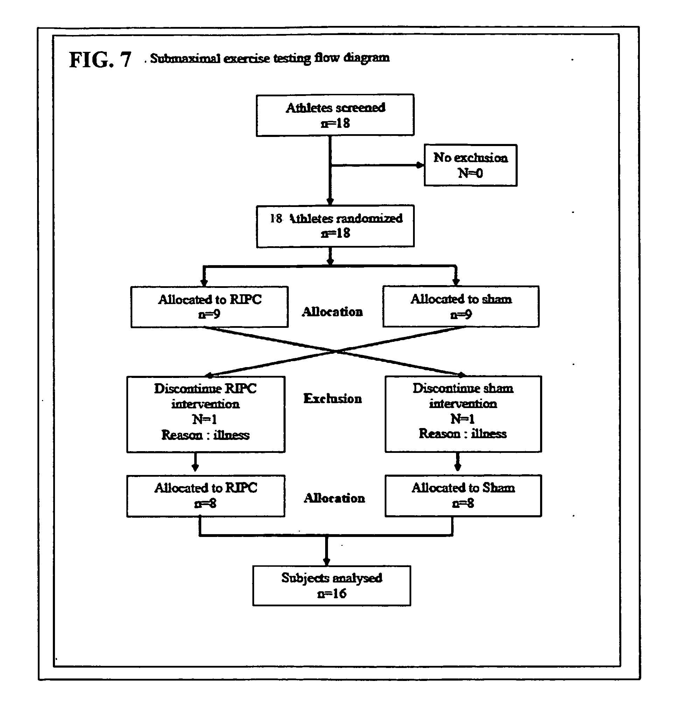

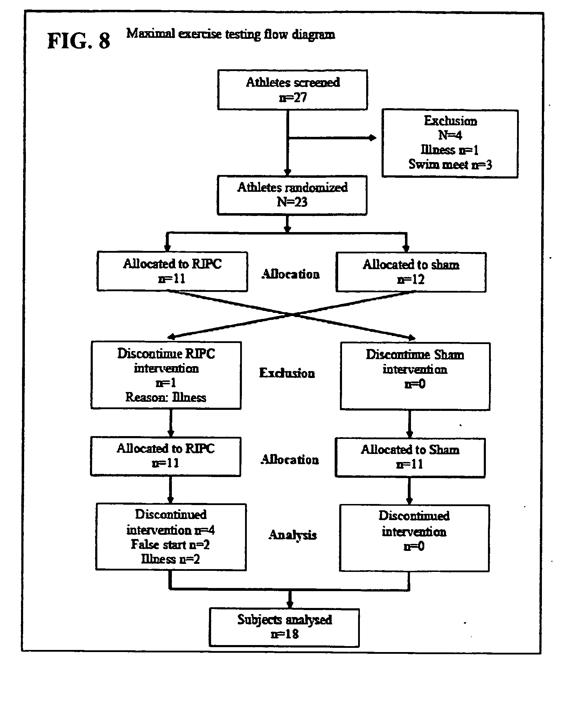

The randomisation list was computer generated. The randomisation codes were sealed in opaque envelopes and were assigned to the athletes after their enrollment in the study. Subjects were randomized to receive either four five-minute cycles of upper limb ischemia interspaced with five minutes of reperfusion, or a sham procedure. Ischemia was achieved by inflating a blood-pressure cuff to a pressure of 15 mmHg greater than measured systolic arterial pressure. For the sham procedure, the blood pressure cuff was inflated to only 10 mmHg. The 'reperfusion' period consisted of five minutes after full cuff deflation. On the subsequent study date, separated by one week from the previous one, the subjects were submitted to the intervention they had not received. All study investigators and participants were blinded to treatment assignment for the duration of the study. The group allocation was not revealed to the investigators until the end of the statistical analysis and the athletes were not told which inflation could be beneficial to their swimming performance. Before the different swimming performance tests, the swimmers swam their normal warm-up and it was the same on each test occasion. FIGs. 7 and 8 show the details of patient recruitment and randomisation for both the submaximal and maximal exercise test protocols (see below).

The submaximal exercise swimming protocol has been previously reported (ref. 11, 28). The test was conducted in a long course pool (i.e. 50 meters in length). Before the swimming test, participant's weight and height was measured (ref. 12). Each submaximal swim test consisted of seven sequential 200 meters swims. Each 200 meters swim commenced at six minute intervals and began from a push start. The coach calculated the required speed for each 200 meters swim prior to the test and the participants were informed of these target speeds before the test began. Each target speed was based on a fixed percentage of the participant's best time. For example, the first 200 meters were swum at a speed that would result in a time equal to the individual's best time + 35 seconds. Thereafter, each subsequent 200 meters were completed approximately five seconds faster than the preceding swim. Time, heart rate (RS 800, Polar Electro Inc.), stroke rate and blood lactate were measured and recorded for each swimming increment. Blood samples were obtained from a finger stick and analyzed using the Lactate ProTM LT-1710 Analyzer (Arkray Lt., Japan) approximately two minutes after the completion of each swim. The swimmers were asked to swim the performance test in their best stroke style (e.g. freestyle, backstroke, breaststroke, fly, individual medley).

The maximal swimming performance test was also completed in a long course pool. The swimmers swam there preferred swim length, 100 meters (n= 16) or 200 meters (n=2), using their best stroke style at 100% effort. Blood lactates were measured before and after the test. Time, blood lactate and stroke rate were also measured. Blood samples were obtained from a finger stick and analyzed using the Lactate ProTM LT-1710 Analyzer (Arkray Lt., Japan) approximately two minutes after the completion of the swim. The maximal swimming performance testing was done either at a University swimming competition or in a 'time trial' competition setting. In both cases warm-up procedures were identical in both test conditions. The primary endpoint of the submaximal study was an improvement in the critical velocity, defined as the extrapolated intersection between the maximal heart rate and swimming velocity of preconditioned subjects during incremental exercise testing. The primary end point for the maximal exercise test was the swim time. Our secondary endpoints were change in peak blood lactate level and change in stroke rate.

All animal protocols relating to the Langendorff protocol were approved by the Animal Care and Use Committee of the Hospital for Sick Children in Toronto and conformed with the Guide for the Care and Use of Laboratory Animals published by NIH (NIH publication N0. 85- 23, revised 1996). Blood samples (30 mls) were obtained before and after RIPC in nine of the junior national level swimmers and four control healthy non-athlete subjects. Our experimental method has been described in detail in a previous publication (ref. 21). Briefly, the blood was collected in heparinized tubes and immediately put on ice prior to centrifugation at 3000 rpm for 20 minutes at room temperature. The plasma fraction was carefully removed without disturbing the buffy coat and it was placed in a 12-14 KD dialysis tubing (Spectrapor, USA) and dialysed against a 10 or 20 fold volume of Krebs- Henseleit solution. For use in the Langendorff system, the dialysate was made isotonic by adjusting the salts: NaCl 130 mmol/L, MgSO4·7H2O 0·5 mmol/L, KCL 4·7 mmol/L, CaCl2 1·0 mmol/L, KH2PO4 1·2 mmol/L, HEPES 20 mmol/L in a 10X Krebs-Henseleit buffer stock. Finally the pH was adjusted to 7·4 by addition of sodium bicarbonate (NaHCO3) and glucose. The dialysate was equilibrated to 37°C and oxygenated for 20 min prior to use in the mouse Langendorff. The mice were anesthetized with pentobarbital (60 mg/kg ip) and the hearts were excised, chilled with cold saline, and cannulated under a microscope via the aorta. The hearts were then perfused in the Langendorff mode with modified Krebs-Ringer buffer at 37°C consisting (in mM) of 119 NaCl, 4·8 KCl, 1·3 CaCl2, 1·2 KH2PO4, 1·2 MgSO4, 25 NaHCO3. A water filled latex balloon was placed in the left ventricular cavity via the mitral valve. This balloon was connected to a pressure transducer and kept a constant pressure of 6 mmHg. The peak left ventricular developed pressure (LVP) was continuously monitored. Each heart underwent an initial 20 minutes stabilization period. The hearts were then perfused with the human dialysate, and subsequently subjected to 30 minutes of global zero-flow ischemia, followed by 60 minutes of post-ischemia reperfusion. The hemodynamic measurements, including heart rate (HR), peak left ventricular pressure (LVP), the maximum rate of pressure increase (+dP/dtmax), the maximum rate of pressure decrease (-dP/dtmax), and the coronary flow were recorded throughout the experiment. After completion of the Langendorff protocol, the hearts were frozen with liquid nitrogen after being submerged in a high potassium solution and then stored at -80°C. The hearts are then put into a slicer matrix and cut into one to two millimeters thick slices. The slices were immersed in a 1·25% 2, 3, 5-triphynyl-tetrazolium chloride (TTC) (sigma T8877) and kept in a waterbath at 40°C for 15 minutes. The slices were then fixed in 10% formalin. The fixed slices were photographed and scanned using photoshop and the percentage of infarcted area was expressed as a ratio to the total left ventricular area.

### Statistical analysis

Data are described as means with standard deviations, median with minimum and maximum values and frequencies as appropriate. Differences between exercise performances between real and sham RIPC were assessed in paired t-tests. Difference in infarct size between mice hearts perfused with elite athletes' dialysate and normal controls' dialysate were assessed using Student's t-tests. The effect of potential confounders including subject: age, gender, personal best time, FINA ranking, competitive level, stroke and order of randomization were assessed in linear regression models adjusted for repeated measures through a compound symmetry covariance structure. All statistical analyses were performed using SAS statistical software v9.1 (The SAS Institute, Cary NC).

### Results

A total of 27 athletes were eligible for randomisation. The submaximal exercise test was completed by 16 athletes (FIG. 7) and 22 subjects completed the maximal exercise intervention (FIG. 8) from 4 different swimming teams across Canada (Vancouver, Toronto, Guelph). Three athletes were unable to participate in the maximal performance testing because of a conflict with their competition schedule. Six swimmers were excluded from the analysis because of false starts and/or illnesses on the second study day. Subjects with false starts were excluded 238 from the study because by not starting on time, they modified significantly their swim time independent of actual performance. Table 6 provides the characteristics of the elite swimmers included in the study analysis of the submaximal exercise protocol and Table 7 describes the characteristics for those completing the maximal exercise protocol. Table 8 demonstrates the difference in patient characteristics between the 2 intervention days for the maximal exercise protocol. There were no protocol deviations. Submaximal incremental swimming test results: We did not demonstrate any significant effect of RIPC on any of the indicators of submaximal exercise performance (Table 9). In particular, there was no effect on our primary end-point, critical velocity, or maximal heart rate. The velocity achieved at a lactate concentration of 4 mmol/L was also unaffected.

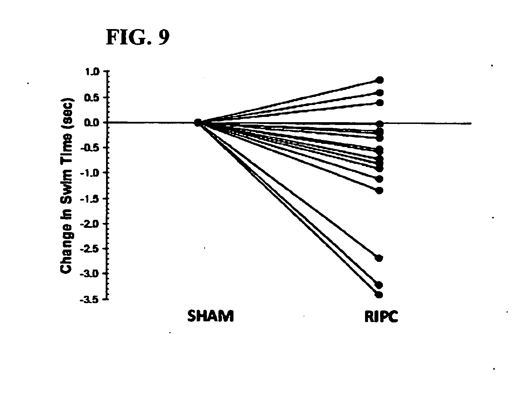

Maximal competitive swimming test results: RIPC was associated with an improvement in competitive swim times (FIG. 9). Table 10 shows the effect of RIPC on the indicators of maximal performance. RIPC was associated with a significant improvement in competitive swim time for 100 meters of, on average, 0.7 seconds (66·98±21·28 sec vs. 66·28±21·08 sec, p=0·04) and a superior swim time relative to personal best time (+4·66±3·76% vs. +3·55±3·31%, p=0·02). Moreover, this improvement in swim time was not achieved at the expense of increased lactate production or increased heart rate. However, there was a trend towards an increase in number of strokes (p=0· 12) and no increase in heart rate (n=5) (180±11 bpm, 180±8 bpm, p=0·96). RIPC was also associated with a smaller mean absolute difference compared with personal best swim time and with a higher average FINA point (627±69 vs. 650±64, p=0·01) (Table 10). No factors were found to be confounders of the association between race time and RIPC stimulus. On a sub-analysis, the subjects' competitive level (national vs. international) did not affect the association between RIPC and improved maximal performance (+0·006 sec, p=0·52).

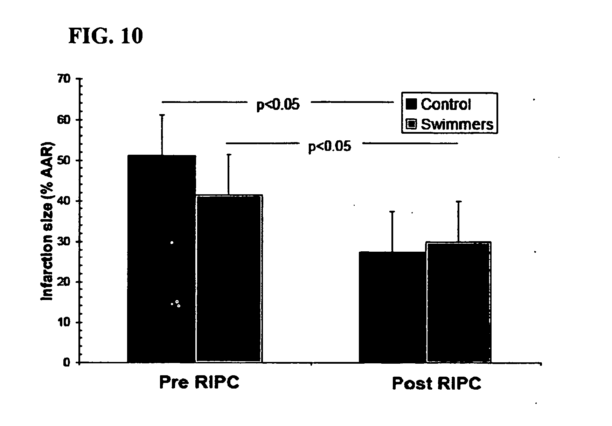

Langendorff experiments: Athletes and control subjects underwent blood sampling before and after RIPC. Comparing pre-RIPC dialysate to post RIPC dialysate, the infarct size was reduced from 51·2 ± 10·9 % to 27 ± 2·2 % (p=0·06) for the control subjects and reduced from 41·3 ± 5·2 % to 29·7 ± 2·7 % (p=0·05) in the swimmers (FIG. 10). There was no significant difference between the control group and elite athletes (p=0·40 and p=0·68 for pre and post RIPC, respectively). However, left ventricular generated pressure was higher from 25 to 60 min of reperfusion in mice hearts perfused with post-RIPC dialysate from the swimmers (89·9 ± 2·1 mmHg, 83·5 ± 2·9 mmHg respectively, p=0·04). No other endpoints were significantly influenced by the RIPC intervention. There were no adverse events or side effects associated with the real or sham remote preconditioning intervention.

### Discussion

In this study, RIPC was not associated with an improvement in incremental submaximal exercise, but was associated with significantly improved maximal performance in elite swimmers. Our hypothesis was that intense exercise represents a physiologic form of ischemic injury, and therefore may be amenable to modification by ischemic preconditioning. In this study, we used our simple method of remote ischemic preconditioning, by transient upper limb ischemia, in a group of elite swimmers. Swimming is an unusual sport in which controlled ventilation and a very high rate of energy turnover leads to a marked reduction in PaO₂ with measured O₂ saturations falling to between 80-85% in highly trained individual (ref. 10) and therefore represents an ideal model to test the effects of RIPC. Indeed, swimming performance is thought to be, at least in part, limited by exercise induced arterial hypoxemia (ref. 28). Associated with this is a fall in arterial pH and a substantial rise in venous lactate (ref. 31), reflecting tissue hypoxemia and metabolic acidosis. We hypothesized that RIPC might modify skeletal muscle tolerance to this tissue hypoxia, thereby improving maximal and submaximal exercise performance. RIPC is a phenomenon that is known to protect tissues against ischemia and reperfusion injury that occurs as a result of cessation of blood flow to a tissue bed, such as during cardiac surgery (ref. 29), or myocardial infarction (ref. 30). As such, it recapitulates the effects of local preconditioning, albeit in a more facile and clinically relevant way. In a later study in human exercise performance, 'local' preconditioning of each leg was shown to improve peak oxygen consumption during bicycle exercise testing in normal healthy subjects (ref. 26). The current study used transient upper arm ischemia as the stimulus of 'remote' preconditioning. We have recently shown that RIPC induced by transient limb ischemia leads to release of a cardioprotective factor, or factors, into the bloodstream of animals and humans (ref. 21). The effect of this factor was manifest as increased tolerance to myocardial ischemia-reperfusion injury in a rabbit Langendorff model. In this study, we confirmed that this humoral mechanism persists in elite swimmers, and presumably contributes or explains the improved tolerance to exercise induced hypoxemia and acidosis during extreme exercise in the swimmers, where all muscle groups are being used. Interestingly, there was no significant effect on incremental submaximal exercise tolerance in the same individuals. While our study was not designed to explore subcellular mechanisms, it is possible that the difference observed is related to differences in the pathways of energy utilization during submaximal exercise and at maximal exertion. During submaximal exercise, energy is produced mainly by the aerobic oxidative pathway, whereas during maximal performance, energy is produced by the breakdown of phosphocreatinine but also by the anaerobic glycolytic pathway (ref. 31) in addition to the aerobic oxidative system. It is known from performance models that predicted exercise capacity is determined by the capacity to produce energy (ATP) by different metabolic pathways (ref. 10). Interestingly, in vivo studies have shown that IPC leads to opening of mitochondrial ATP sensitive K-channels and uncoupling of oxidative phosphorylation (ref. 14). As a result, we speculate that RIPC allows for faster uptake of Acetyl- CoA (a breakdown product of glycolysis) by mitochondria, thus maintaining lactate accumulation at a metabolically acceptable level and contributing aerobically-generated ATP for exercise. Our observations of a trend toward a higher stroke rate and improved swimming time without a change in post-swim blood lactate level support this hypothesis.

The 0·70 second reduction in time was not only statistically significant, but also of major physiologic and competitive significance to the , representing a 1·11 % improvement in swim time. It has previously been suggested that an improvement of 0·4% in competition performance is a 'competitively significant' change (ref. 16). Such improvements are usually generated by a structured training program. In elite swimmers, the relationship between the training regimen and the competitive performance is well described (ref. 27). From the test data, our observed improvement in competitive swim time of 0·7 seconds would represents, on average, two years of training in these highly trained individuals (ref. 16).

### Example 3: Exercise tolerance in subjects having chronic stable angina.

### Background

Remote ischemic preconditioning (RIPC), induced by transient ischemia and reperfusion (IR) of the limb, has been shown to protect against IR injury following prolonged ischemia in the heart, kidney and brain. Recently RIPC performed prior to elective PCI was shown to decrease ischemic pain, troponin release ST-segment change, and subsequent adverse cardiovascular outcomes at 6 months (Circulation 2009;119(6):820-7). In this study we examined the effect of RIPC on exercise tolerance in patients with chronic stable ischemic heart disease.

### Methods

Sixty patients were treated for 5 consecutive days with RIPC consisting of four 5-minute cycles of upper limb ischemia (using a blood pressure cuff inflated to 15mmHg suprasystolic pressure) interspaced with 5 minutes of reperfusion. Ischemic tolerance before and immediately after the final RIPC was evaluated using treadmill exercise testing. Measurements for Hs-CRP and BNP were also taken.

### Results

There was a significant increase in the braces grade after preconditioning, from grade II to III (P<0.05), along with improved maximal performance 8.25±2.1 vs. 9.67±2.2 Mets, P<0.05), and exercise time (P<0.05). Going along with this, there was a reduction in ST segments depression in patients improved significantly more after RIPC (values P<0.05).

### Conclusion

RIPC delivered daily for 5 days leads to a significant improvement in exercise capacity and evidence of improved myocardial tolerance in patients with chronic stable angina.

### REFERENCES

1. Massie BM, Shah NB (1997) Evolving trends in the epidemiologic factors of heart failure: rational for preventive strategies and comprehensive disease management. Am Heart J 133:703-712
2. Laskey WK, Beach D. Frequency and clinical significance of ischemic preconditioning during percutaneous coronary intervention. J Am Coll Cardiol 2003;42:998 -1003.
3. Teoh LK, Grant R, Hulf JA, Pugsley WB, Yellon DM. The effect of preconditioning (ischemic and pharmacological) on myocardial necrosis following coronary artery bypass graft surgery. Cardiovasc Res, 2002;53: 175-80.
4. Przyklenk K, Bauer B, Ovize M, Kloner RA, Whittaker P. Regional ischemic 'preconditioning' protects remote virgin myocardium from subsequent sustained coronary occlusion. Circulation 1993;87:893-9.
5. Cheung MM. Kharbanda RK. Konstantinov IE. Shimizu M. Frndova H. Li J. Holtby HM. Cox PN. Smallhorn JF. Van Arsdell GS. Redington AN. Randomized controlled trial of the effects of remote ischemic preconditioning on children undergoing cardiac surgery: first clinical application in humans. Journal of the American College of Cardiology. 47(11):2277-82, 2006 Jun 6.
6. Paterson, D. J., G. A. Wood, et al. (1986). "The entrainment of ventilation frequency to exercise rhythm." Journal of Applied Physiology, 55(5): 530-7.
7. Wells GD, Plyley M, Thomas S, Goodman L, Duffin J. Effects of concurrent inspiratory and expiratory muscle training on respiratory and exercise performance in competitive swimmers. European Journal of Applied Physiology. 2005 94(5-6): 527-40.
8. Sharp, R. L., Williams, D., Bevan, L. (1991). "Effects of controlled frequency breathing during exercise on blood gases and acid-base balance." International Journal of Sports Medicine, 12: 62-65.
9. Craig AB Jr Breath holding during the turn in competitive swimming. Medicine & Science in Sports & Exercise. 18(4):402-7, 1986 Aug.
10. Noakes T. Physiological models to understand exercise fatigue and the adaptations that predict or enhance athletic performance. Scand J Med Sci Sports 2000: 10: 123-145.
11. Pyne, D. B., Maw, G., & Goldsmith, W. (2000). Protocols for the physiological assessment of swimmers. In C. J. Gore (Ed.), Physiological tests for elite athletes (pp. 372 - 382). Champaign, IL: Human Kinetics.
12. Wells GD, Schneiderman-Walker J, Plyley M. Normal physiological characteristics of elite swimmers. Pediatric Exercise Science. 2006 17: 30-52.
13. Grimston, S. K. and J. G. Hay (1986). "Relationships among anthropometric and stroking characteristics of college swimmers." Medicine and Science in Sports and Exercise, 18(1): 60-8.
14. Fryer RM, Eells JT, Hsu AK, Henry MM, Gross GJ. Ischemic preconditioning in rats: role of mitochondrial K(ATP) channel in preservation of mitochondrial function. Am J Physiol Heart Circ Physiol 2000;278:H305-H312.
15. Robazza C. Pellizzari M. Bertollo M. Hanin YL. Functional impact of emotions on athletic performance: comparing the IZOF model and the directional perception approach. [Comparative Study. Journal Article] Journal of Sports Sciences. 26(10):1033-47, 2008 Aug.
16. Anderson M. Hopkins W. Roberts A. Pyne D. Ability of test measures to predict competitive performance in elite swimmers. [Journal Article] Journal of Sports Sciences. 26(2):123-30, 2008 Jan 15.
17. Chatard JC. Wilson B. Effect of fastskin suits on performance, drag, and energy cost of swimming. [Journal Article. Research Support, Non-U.S. Gov't] Medicine & Science in Sports & Exercise. 40(6):1149-54, 2008 Jun.
18. Vinten-Johansen J. Postconditioning: a mechanical maneuver that triggers biological and molecular cardioprotective responses to reperfusion. Heart Failure Review 2007; 12:235-244.
19. Downey JM, Davis AM, Cohen MV. Signaling pathways in ischemic preconditioning. Heart Failure Reviews. 2007; 12:181-188.
20. Kharbanda RK, Nielsen TT, Redington AN. Translation of remote ischaemic preconditioning into clinical practice. Lancet 2009; 374:1557-1565.
21. Shimizu M, Konstantinov IE, , Kharbanda RK, Cheung MH, Redington AN. Effects of intermittent lower limb ischemia on coronary blood flow and coronary resistance in pigs. Acta Physiol (Oxf) 2007; 190:103-109.
22. Hausenloy DJ, Mwamure PK, Venugopal V, Harris J, Barnard M, Grundy E, Ashley E, Vichare S, Di Salvo C, Kolvekar S, Hayward M, Keogh B, MacAllister RJ, Yellon DM. Effect of remote ischaemic preconditioning on myocardial injury in patients undergoing coronary artery bypass graft surgery: a randomized controlled trial. Lancet. 2007 Aug 18; 370:575-9.
23. Ali ZA, Callaghan CJ, Lim E, Ali AA, Nouraei R, Akthar AM, Boyle JR, Varty K, Kharbanda RK, Dutka DP, Gaunt ME. Remote ischemic preconditioning reduces myocardial and renal injury after elective abdominal aortic aneurysm repair: arandomized controlled trial. Circulation 2007; 116:198-105.
24. Hoole SP, Heck PM, Sharples L, Khan SN, Duehmke R, Densem CG, Clarke SC, Shapiro LM, Schofield PM, O'Sullivan M, Dutka DP. Cardiac Remote Ischemic Preconditioning in Coronary Stenting (CRISP Stent) Study: a prospective, randomized control trial. Circulation 2009; 119:820-827.
25. Bøtker HE, Kharbanda R, Schmidt MR, Bøttcher M, Kaltoft AK, Terkelsen CJ, Munk K, Andersen NH, Hansen TM, Trautner S, Lassen JF, Christiansen EH, Krusell LR, Kristensen SD, Thuesen L, Nielsen SS, Rehling M, Sørensen HT, Redington AN, Nielsen TT. Remote ischaemic conditioning before hospital admission, as a complement to angioplasty, and effect on myocardial salvage in patients with acute myocardial infarction: a randomized trial. Lancet 2010; 37:727-734.
26. Groot P, Thijssen DH, Sanchez M, Ellenkamp R, Hopman M. Ischemic preconditioning improves maximal performance in humans. Eur J Appl Physiol 2009; 108:1195-1202.
27. Mujika L., Padilla S, Pyne D. Swimming performance changes during the final 3 weeks of training leading to the Sydney 2000 Olympic. International Journal of Sport Medicine 2002; 23:582-587.
28. Wells GD, Plyley M, Thomas S, Goodman L, Duffin J. Effects of concurrent inspiratory and expiratory muscle training on respiratory and exercise performance in competitive swimmers.. Eur J Appl Physiol 2005; 94:527-540.
29. Chaturvedi RR, Lincoln C, Gothard JW, Scallan MH, White PA, Redington AN, Shore DF. Left ventricular dysfunction after open repair of simple congenital heart defects in infants and children: quantitation with the use of a conductance catheter immediately after bypass. J Thorac Cardiovasc Surg 1998; 115:77-83.
30. Yellon DM, Hausenloy DJ. Myocardial reperfusion injury. N Engl J Med 2007; 357:1121-1135.
31. Wells GD, Selvadurai H, Tein I. Bioenergetic provision of energy for muscular activity. Paediatr Respir Rev 2009; 10:83-90.
32. Murry CE, Jennings RB, Reimer KA. Preconditioning with ischemia: a delay of lethal cell injury in ischemic myocardium. Circulation 1986; 74:1124-1136.

The foregoing written specification is considered to be sufficient to enable one ordinarily skilled in the art to practice the invention. The present invention is not to be limited in scope by examples provided, since the examples are intended as mere illustrations of one or more aspects of the invention. Other functionally equivalent embodiments are considered within the scope of the invention. Various modifications of the invention in addition to those shown and described herein will become apparent to those skilled in the art from the foregoing description. Each of the limitations of the invention can encompass various embodiments of the invention. It is, therefore, anticipated that each of the limitations of the invention involving any one element or combinations of elements can be included in each aspect of the invention. This invention is not limited in its application to the details of construction and the arrangement of components set forth or illustrated in the drawings. The invention is capable of other embodiments and of being practiced or of being carried out in various ways.

Also, the phraseology and terminology used herein is for the purpose of description and should not be regarded as limiting. The use of "including", "comprising", or "having", "containing", "involving", and variations thereof herein, is meant to encompass the items listed thereafter and equivalents thereof as well as additional items.

**Table 1: Characteristics of the athletes enlisted in the study**

| | | *Athletes* | |
|---|---|---|---|
| | *Demographic* | | |
| | Mean age (years ± SD) | 17.2 ± 1.0 | |
| | Gender (female) | 6 (55%) | |
| | Mean height (cm ± SD) | 175 ± 9 | |
| | Mean weight (Lbs ± SD) | 150 ± 24 | |
| | Mean 1000 points ranking (± SD) | 792 ± 79 | |

| | *Stroke* | | |
|---|---|---|---|
| | Breaststroke | 3 (27%) | |
| | Fly | 2 (18%) | |
| | Individual medley | 1 (9%) | |
| | Freestyle | 4 (36%) | |
| | Backstroke | 1 (9%) | |

| | *Randomization-Study A* | | |
|---|---|---|---|
| | RIPC first week (vs. SHAM first week) | 5 (45%) | |

| | *Randomization-Study. B* | | |
|---|---|---|---|
| | RIPC first week (vs. SHAM first week) | 4 (50%) | |
| | | | |

**Table 2: Summary of results for submaximal exercise test**

| | | | | | |
|---|---|---|---|---|---|
| | | | | | |

| | | ***SHAM*** | ***RIPC*** | ***P*** | |
|---|---|---|---|---|---|
| | | | | | |
| | Critical velocity | 1.40 ± 0.15 | 1.41 ± 0.16 | 0.59 | |
| | Maximal velocity (m/sec) | 1.36 ± 0.12 | 1.35 ± 0.12 | 0.50 | |
| | Maximum heart rate (beats/min) | 214.2 ± 74.8 | 206.0 ± 71.0 | 0.59 | |
| | Heart rate slope | 187.0 ± 9.9 | 188.0 ± 10 | 0.60 | |
| | Velocity at 4mmol/L lactate (m/min) | 1.3 ± 0.1 | 1.3 ± 0.1 | 0.58 | |
| | Maximum lactate (mmol/L) | 13.6 ± 2.4 | 12.9 ± 2.7 | 0.22 | |
| | Stroke rate Slope | 1.1 ± 0.3 | 1.2 ± 0.4 | 0.32 | |
| | | | | | |

**Table 3: Linear regression models adjusted for repeated measures and order of treatment assignment**

| | | | | |
|---|---|---|---|---|
| | | | | |

| | | ***EST (SE)*** | ***p*** | |
|---|---|---|---|---|
| | | | | |
| | Critical velocity | -0.006 (0.008) | 0.46 | |
| | Maximal velocity (m/sec) | 0.005 (0.008) | 0.56 | |
| | Maximum heart rate (beats/min) | 1.000 (1.778) | 0.58 | |
| | Heart rate slope | -8.245 (14.20) | 0.57 | |
| | Velocity at 4mmol/L lactate (m/sec) | -0.005 (0.009) | 0.55 | |
| | Maximum lactate (mmol/L) | -0.664 (0.478) | 0.17 | |
| | SR slope | 0.065 (0.059) | 0.27 | |
| | | | | |

**Table 4: Summary of results for maximal exercise test**

| | | | | | |
|---|---|---|---|---|---|
| | | | | | |

| | | ***SHAM*** | ***RIPC*** | ***p*** | |
|---|---|---|---|---|---|
| | | | | | |
| | Time (sec) | 64.9 ± 3.9 | 64.0 ± 3.7 | 0.05 | |
| | Pre lactate (mmol/L) | 1.67 ± 0.32 | 1.74 ± 0.34 | 0.74 | |
| | Post lactate (mmol/L) | 11.04 ± 1.59 | 11.69 ± 2.80 | 0.51 | |
| | Number of strokes | 28.6 ± 8.8 | 29.6 ± 8.3 | 0.17 | |
| | | | | | |

**Table 5: Linear regression models adjusted for repeated measures and order of treatment assignment**

| | | | | |
|---|---|---|---|---|
| | | | | |

| | | ***EST (SE)*** | ***p*** | |
|---|---|---|---|---|
| | | | | |
| | Time (sec) | -0.929 (0.355) | 0.009 | |
| | Pre lactate (mmol/L) | 0.069 (0.019) | 0.72 | |
| | Post lactate (mmol/L) | 0.650 (0.867) | 0.46 | |
| | Number of strokes | 1.000 (0.612) | 0.11 | |
| | | | | |

**Table 6: Characteristics of the athletes enlisted in the submaximal protocol. IM = individual medley. Remote preconditioning = RIPC. (Updated Study Results)**

| | | *Athletes* | |
|---|---|---|---|
| | *Demographic* | | |
| | Mean age (year ± SD) | 18·8 ± 3·3 | |
| | Gender (female) | 8 (50%) | |
| | Mean height (cm ± SD) | 179·9 ± 10·1 | |
| | Mean weight (Lbs ± SD) | 72·7 ± 10·7 | |
| | Mean 1000 points rauking (± SD) | 899 ± 215 | |

| | *Stroke* | | |
|---|---|---|---|
| | Breaststroke | 2 (13%) | |
| | Fly | 4 (2.5%) | |
| | IM | 2 (13%) | |
| | Freestyle | 7 (44%) | |
| | Backstroke | 1 (5%) | |

| | *Randomisation- Study A* | | |
|---|---|---|---|
| | RIPC first week | 8 (50%) | |

| | *Rondomisation- Study B* | | |
|---|---|---|---|
| | SHAM first week | 8 (50%) | |
| Table 6 Characteristics of the athletes enlisted in the submaximal protocol | | | |

**Table 7: Characteristics of the athletes included in the maximal protocol. IM is the abbreviation for individual medley. Remote preconditioning = RIPC. (Updated Study Results)**

| | | *Athletes* | |
|---|---|---|---|
| | *Demographic* | | |
| | Mean age (year ± SD) | 19·2 ± 2·9 | |
| | Gender (female) | 8 (50%) | |
| | Mean height (cm ± SD) | 180·2 ± 8·0 | |
| | Mean 1000 points ranking (± SD) | 899 ± 215 | |

| | *Stroke* | | |
|---|---|---|---|
| | Breaststroke | 3 (17%) | |
| | Fly | 4 (22%) | |
| | IM | 3 (17%) | |
| | Freestyle | 7 (39%) | |
| | Backstroke | 1 (5%) | |

| | *Rondomisation- Study A* | | |
|---|---|---|---|
| | RIPC first week | 11 (61%) | |

| | *Randomisation- Study B* | | |
|---|---|---|---|
| | SHAM first week | 7 (39%) | |
| Table 7 Characteristics of the athletes enlised in the maximal protocol | | | |

**Table 8: Characteristics of the athletes included in the maximal protocol. SBP represents systolic blood pressure and DBP represents diastolic blood pressure. (Updated Study Results)**

| | | *Sham* | *RIPC* | *p value* | |
|---|---|---|---|---|---|
| | Preceding respiratory illness (n=9) | 1 (11%) | 3 (33%) | 0·58 | |
| | SBP (mmHg) (n=5) | 120±12 | 124±14 | 0·41 | |
| | DBP (mmHg) (n=5) | 65±7 | 64±8 | 0·50 | |
| | Table 8 Characteristics of the athletes in the maximal protocol | | | | |

**Table 9: Effect of RIPC on submaximal exercises performance indicators. Peak velocity (Vpeak) is expressed in meters per second. Critical velocity (Vcrit) is expressed in meters per second. Maximal heart rate (Max HR) is expressed in beats per minute. Heart rate slope (HR slope) is expressed in beats per minute multiplied by seconds, divided by meter. Lactate peak (LA peak) is expressed in mmol/L. SEI represents t swimmer's Swim Efficiency Index. (Updated Study Results)**

| | | *SHAM* | *RIPC* | *P value* | |
|---|---|---|---|---|---|
| | Vpeak (m/sec) | 144±0·15 | 1.44±0.15 | 0·84 | |
| | Vcrit (m/sec) | 1.41±0·15 | 1·41±0·15 | 0·53 | |
| | Max HR (bpm) | 190·1±13·0 | 192·3±13.1 | 0·39 | |
| | HR slope (bpm*sec/m) | 217·4±66.9 | 221·5±73·9 | 0·75 | |
| | LA peak (mmol/L) | 13·0±2·3 | 12·7±2·5 | 0·37 | |
| | SEI mean (index) | 2·61±0·98 | 2·60±0·95 | 0·44 | |
| | Table 9 Effect of RIPC on submaximal exercises performance indicators | | | | |

**Table 10: Effect of RIPC on maximal exercises performance indicators. FINA points = international swimming federation point score. (Updated Study Results)**

| | | *SHAM* | *RIPC* | *P value* | |
|---|---|---|---|---|---|
| | time (s) | 66·98±21·28 | 66·28±21·08 | 0·04 | |
| | Mean absolute difference with best time (s) | +2·83±2·47 | +2·13±1·83 | 0·04 | |
| | Mean relative difference with best time (%) | +4·66±3·76 | +3·55±3·31 | 0·02 | |
| | FINA (/1000) (n=21) | 627±69 | 650±64 | 0·01 | |
| | Lactate (mmol/L) | 12·3±2·0 | 12·8±2·4 | 0·38 | |
| | Number of strokes | 20·9±9·3 | 21·5±9·5 | 0·12 | |
| | Table 10 Effect of RIPC on maximal exercises performance indicators | | | | |

## Claims

1. A method for enhancing the competitive athletic performance of an athlete in maximal physical activity, the method comprising
performing a remote ischemic preconditioning regimen on the athlete;
and then, within 24 hours after said regimen;
performing the competitive athletic maximal physical activity.

2. The method of claim 1, wherein the remote ischemic preconditioning regimen is performed within 2 hours of the physical activity.

3. The method of claim 1, wherein the remote ischemic preconditioning regimen is performed within 20 minutes of the physical activity.

4. The method of claim 1 to 3, wherein the remote ischemic preconditioning regimen comprises 1, 2, 3, 4, or 5 cycles of supra-systolic pressure and reperfusion.

5. The method of claim 1 to 3, wherein the remote ischemic preconditioning regimen comprises at least four cycles of supra-systolic pressure and reperfusion.

6. The method of any one of the preceding claims, wherein the remote ischemic preconditioning regimen comprises cycles which include a period of supra-systolic pressure which has a duration of between 1 and 20 minutes.

7. The method of any one of the preceding claims, wherein the remote ischemic preconditioning regimen includes a period of reperfusion which has a duration of between 1 and 20 minutes.

8. The method of any one of the preceding claims, wherein the remote ischemic preconditioning regimen comprises cycles of 5 minutes of supra-systolic pressure and 5 minutes of reperfusion.

9. The method of any one of the preceding claims, wherein the supra-systolic pressure is a pressure that is at least 15 mmHg above systolic pressure.

10. The method of any one of the preceding claims, wherein the remote ischemic preconditioning regimen is performed on an upper limb.

11. The method of any one of claims 1-9, wherein the remote ischemic preconditioning regimen is performed on a lower limb.

12. Use of a device comprising a contractable cuff to perform a remote ischemic preconditioning regimen in a method comprising
performing a remote ischemic preconditioning regimen on the athlete;
and then, within 24 hours after said regimen;
performing the competitive athletic maximal physical activity.

13. Use as claimed in claim 12 wherein the device comprises
a cuff configured to retract about a remote location of a subject;
an actuator connected to the cuff and that, when actuated, causes the cuff to contract about the remote location of the subject to reduce blood flow therethrough; and
a controller that controls the actuator.

14. Use as claimed in claim 13, wherein the controller controls the actuator to operate according to a treatment protocol that includes one or a plurality of sequentially actuated treatment cycles, each treatment cycle comprising
cuff actuation, during which the actuator contracts the cuff about the remote location of the subject to a pressure above systolic pressure to occlude blood flow through the remote location;
an ischemic duration, during which the actuator maintains the cuff contracted about the remote location at a set point above systolic pressure to occlude blood flow through the remote location;
cuff release, during which the actuator releases the cuff to allow blood flow through the remote location; and
a reperfusion duration, during which the cuff is maintained in an at least partially relaxed state to allow blood flow through the remote location.

15. Use as claimed in any one of claims 12 to 14, wherein the cuff configured to retract about a remote location of a subject is comprised within an athletic garment.

16. Use as claimed in claim 15, wherein the garment comprises two or more cuffs, each configured to retract about a remote location of a subject.

## Patentansprüche

1. Verfahren zum Verbessern der athletischen Wettbewerbsleistung eines Athleten bei maximaler physischer Aktivität, wobei das Verfahren aufweist
Durchführen einer ischämischen Fernpräkonditionierungskur an dem Athleten; und
anschließend, innerhalb von 24 Stunden nach der Kur, Durchführen der wettbewerblichen, athletischen, maximalen physischen Aktivität.

2. Verfahren nach Anspruch 1, bei dem die ischämische Fernpräkonditionierungskur innerhalb von 2 Stunden nach der physischen Aktivität durchgeführt wird.

3. Verfahren nach Anspruch 1, bei dem die ischämische Fernpräkonditionierungskur innerhalb von 20 Minuten nach der physischen Aktivität durchgeführt wird.

4. Verfahren nach Anspruch 1 bis 3, bei dem die ischämische Fernpräkonditionierungskur 1, 2, 3, 4 oder 5 Zyklen von supra-systolischem Druck und Reperfusion umfasst.

5. Verfahren nach Anspruch 1 bis 3, bei dem die ischämische Fernpräkonditionierungskur zumindest vier Zyklen an supra-systolischem Druck und Reperfusion umfasst.

6. Verfahren nach einem der vorhergehenden Ansprüche, bei dem die ischämische Fernpräkonditionierungskur Zyklen aufweist, die eine Zeitdauer mit supra-systolischem Druck umfassen, die eine Dauer zwischen 1 und 20 Minuten aufweisen.

7. Verfahren nach einem der vorhergehenden Ansprüche, bei dem die ischämische Fernpräkonditionierungskur eine Zeitdauer der Reperfusion aufweist, die eine Dauer zwischen 1 und 20 Minuten aufweist.

8. Verfahren nach einem der vorhergehenden Ansprüche, bei dem die ischämische Fernpräkonditionierungskur Zyklen von 5 Minuten an supra-systolischem Druck und 5 Minuten an Reperfusion umfasst.

9. Verfahren nach einem der vorhergehenden Ansprüche, bei dem der supra-systolische Druck ein Druck ist, der zumindest 15 mmHg über dem systolischen Druck liegt.

10. Verfahren nach einem der vorhergehenden Ansprüche, bei dem die ischämische Fernpräkonditionierungskur an einer oberen Extremität durchgeführt wird.

11. Verfahren nach einem der Ansprüche 1 bis 9, bei dem die ischämische Fernpräkonditionierungskur an einer unteren Extremität durchgeführt wird.

12. Verwendung einer Einrichtung aufweisend eine zusammenziehbare Manschette, um eine ischämische Fernpräkonditionierungskur in einem Verfahren durchzuführen, aufweisend:
Durchführen einer ischämischen Fernpräkonditionierungskur an dem Athleten; und
anschließend, innerhalb von 24 Stunden nach der Kur,
Durchführen der wettbewerblichen, athletischen,
maximalen physischen Aktivität.

13. Verwendung nach Anspruch 12, bei der die Einrichtung aufweist :
eine Manschette, die ausgebildet ist, um sich um einen entfernten Ort eines Subjektes einzuziehen;
einen Aktuator, der mit der Manschette verbunden ist und der, wenn er betätigt wird, erwirkt, dass sich die Manschette um einen entfernten Ort des Subjektes einzieht, um einen Blutfluss dort hindurch zu reduzieren; und
ein Steuergerät, das den Aktuator steuert.

14. Verwendung nach Anspruch 13, bei der das Steuergerät den Aktuator steuert, um gemäß einem Behandlungsprotokoll zu wirken, das einen oder eine Vielzahl an sequentiell betätigten Behandlungszyklen umfasst, wobei jeder Behandlungszyklus umfasst:
Manschettenbetätigung, während welcher der Aktuator die Manschette um einen entfernten Ort des Subjektes auf einen Druck oberhalb des systolischen Drucks einzieht, um einen Blutfluss durch den entfernten Ort zu okkludieren;
eine ischämische Dauer, während welcher der Aktuator die Manschette um den entfernten Ort an einem eingestellten Punkt oberhalb des systolischen Drucks eingezogen hält, um den Blutfluss durch den entfernten Ort zu okkludieren;
Manschettenlösen, während welchem der Aktuator die Manschette löst, um einen Blutfluss durch den entfernten Ort zu gestatten; und
eine Reperfusionsdauer, während welcher die Manschette in zumindest einem teilweise entspannten Zustand gehalten wird, um Blutfluss durch den entfernten Ort zu gestatten.

15. Verwendung nach einem der Ansprüche 12 bis 14, bei der die Manschette, die ausgebildet ist, um sich um einen entfernten Ort eines Subjektes einzuziehen, in einem athletischen Kleidungsstück umfasst ist.

16. Verwendung nach Anspruch 15, bei der das Kleidungsstück zwei oder mehr Manschetten aufweist, die jeweils ausgebildet sind, um sich um einen entfernten Ort eines Subjektes einzuziehen.

## Revendications

1. Procédé d'amélioration de la performance sportive en compétition d'un athlète au maximum de son activité physique, le procédé comprenant :
la réalisation d'un régime de préconditionnement ischémique distant sur l'athlète ;
puis, au cours des 24 heures qui. suivent ledit régime ;
la réalisation de l'activité physique maximale sportive en compétition.

2. Procédé selon la revendication 1, dans lequel le régime de préconditionnement ischémique distant est réalisé dans les deux heures de l'activité physique.

3. Procédé selon la revendication 1, dans lequel le régime de préconditionnement ischémique distant est réalisé dans les 20 minutes de l'activité physique.

4. Procédé selon les revendications 1 à 3, dans lequel le régime de préconditionnement ischémique distant comprend 1, 2, 3, 4, ou 5 cycles de pression supra-systolique et de reperfusion.

5. Procédé selon les revendications 1 à 3, dans lequel le régime de préconditionnement ischémique distant comprend au moins quatre cycles de pression supra-systolique et de reperfusion.

6. Procédé selon l'une quelconque des revendications précédentes, dans lequel le régime de préconditionnement ischémique distant comprend des cycles qui comprennent une période de pression supra-systolique dont la durée est comprise entre 1 et 20 minutes.

7. Procédé selon l'une quelconque des revendications précédentes, dans lequel le régime de préconditionnement ischémique distant comprend une période de reperfusion dont la durée est comprise entre 1 et 20 minutes

8. Procédé selon l'une quelconque des revendications précédentes, dans lequel le régime de préconditionnement ischémique distant comprend des cycles de 5 minutes de pression supra-systolique et de 5 minutes de reperfusion.

9. Procédé selon l'une quelconque des revendications précédentes, dans lequel la pression supra-systolique est une pression qui est supérieure d'au moins 15 mmHg à la pression systolique.

10. Procédé selon l'une quelconque des revendications précédentes, dans lequel le régime de préconditionnement ischémique distant est réalisé sur un membre supérieur.

11. Procédé selon l'une quelconque des revendications 1 à 9, dans lequel le régime de préconditionnement ischémique distant est réalisé sur un membre inférieur.

12. Utilisation d'un dispositif comprenant un brassard pouvant se contracter pour réaliser un régime de préconditionnement ischémique distant dans un procédé comprenant :
la réalisation d'un régime de préconditionnement ischémique distant sur l'athlète ;
puis, au cours des 24 heures qui suivent ledit régime ;
la réalisation de l'activité physique maximale sportive en compétition.

13. Utilisation selon la revendication 12, dans laquelle le dispositif comprend :
un brassard configuré pour se rétracter autour d'un emplacement distant d'un sujet ;
un actionneur connecté au brassard et qui, lorsqu'il est actionné, entraîne la contraction du brassard autour de l'emplacement distant du sujet pour réduire le flux sanguin à travers celui-ci ; et
un organe de commande qui commande l'actionneur.

14. Utilisation selon la revendication 13, dans laquelle l'organe de commande commande à l'actionneur de fonctionner selon un protocole de traitement qui comprend un cycle ou une pluralité de cycles de traitement actionnés séquentiellement, chaque cycle de traitement comprenant :
l'actionnement du brassard, durant lequel l'actionneur entraîne la contraction du brassard autour de l'emplacement distant du sujet à une pression supérieure à la pression systolique pour empêcher la circulation du flux sanguin à travers l'emplacement distant ;
une durée d'ischémie, durant laquelle l'actionneur maintient le brassard dans un état contracté autour de l'emplacement distant du sujet à un point défini au-dessus de la pression systolique pour empêcher la circulation du flux sanguin à travers l'emplacement distant ;
le relâchement du brassard, durant lequel l'actionneur entraîne le relâchement du brassard pour permettre la circulation du flux sanguin à travers l'emplacement distant ; et
une durée de reperfusion, durant laquelle le brassard est maintenu dans un état au moins partiellement relâché pour permettre la circulation du flux sanguin à travers l'emplacement distant.

15. Utilisation selon l'une quelconque des revendications 12 à 14, dans laquelle le brassard configuré pour se rétracter autour d'un emplacement distant est compris dans un vêtement de sport.

16. Utilisation selon la revendication 15, dans laquelle le vêtement comprend deux brassards ou plus, chacun étant configuré pour se rétracter autour d'un emplacement distant d'un sujet.
